(19) Europäisches Patentamt
European Patent Office
Office européen des brevets

(11) EP 2 947 157 B1

(12) **EUROPEAN PATENT SPECIFICATION**

(45) Date of publication and mention of the grant of the patent:
**13.09.2017 Bulletin 2017/37**

(21) Application number: **14740752.2**

(22) Date of filing: **16.01.2014**

(51) Int Cl.:
*C12Q 1/68* (2006.01) *G01N 33/48* (2006.01)
*G01N 33/53* (2006.01) *G01N 37/00* (2006.01)
*C12N 5/0735* (2010.01) *C12N 5/074* (2010.01)
*C12N 5/0775* (2010.01)

(86) International application number:
**PCT/JP2014/051316**

(87) International publication number:
**WO 2014/112655 (24.07.2014 Gazette 2014/30)**

(54) **METHOD FOR IDENTIFYING CELLS**

VERFAHREN ZUR IDENTIFIZIERUNG VON ZELLEN

PROCÉDÉ D'IDENTIFICATION DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.01.2013 JP 2013005594**

(43) Date of publication of application:
**25.11.2015 Bulletin 2015/48**

(73) Proprietor: **Universal Bio Research Co., Ltd.**
**Matsudo-shi**
**Chiba 271-0064 (JP)**

(72) Inventors:
- **TAJIMA, Hideji**
**Matsudo-shi**
**Chiba 271-0064 (JP)**
- **GINYA, Harumi**
**Matsudo-shi**
**Chiba 271-0064 (JP)**
- **HATANO, Tomoyuki**
**Matsudo-shi**
**Chiba 271-0064 (JP)**
- **TAKAHASHI, Masaaki**
**Matsudo-shi**
**Chiba 271-0064 (JP)**
- **KARINAGA, Ryouji**
**Matsudo-shi**
**Chiba 271-0064 (JP)**

(74) Representative: **J A Kemp**
**14 South Square**
**Gray's Inn**
**London WC1R 5JJ (GB)**

(56) References cited:
**WO-A1-2012/037456 WO-A1-2012/141324**
**JP-A- 2002 171 973 JP-A- 2006 524 033**

- **KEITH E. SZULWACH ET AL: "Integrating 5-Hydroxymethylcytosine into the Epigenomic Landscape of Human Embryonic Stem Cells", PLOS GENETICS, vol. 7, no. 6, 1 June 2011 (2011-06-01), page e1002154, XP055104552, ISSN: 1553-7390, DOI: 10.1371/journal.pgen.1002154**
- **GULSAH ALTUN ET AL: "DNA methylation in embryonic stem cells", JOURNAL OF CELLULAR BIOCHEMISTRY, 1 January 2009 (2009-01-01), pages n/a-n/a, XP055283530, US ISSN: 0730-2312, DOI: 10.1002/jcb.22374**
- **GRAFODATSKAYA D ET AL: "EBV transformation and cell culturing destabilizes DNA methylation in human lymphoblastoid cell lines", GENOMICS, ACADEMIC PRESS, SAN DIEGO, US, vol. 95, no. 2, 1 February 2010 (2010-02-01), pages 73-83, XP026887165, ISSN: 0888-7543, DOI: 10.1016/J.YGENO.2009.12.001 [retrieved on 2009-12-18]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- RACHEL MICHAELSON-COHEN ET AL: "Genome-Wide De Novo Methylation in Epithelial Ovarian Cancer", INTERNATIONAL JOURNAL OF GYNECOLOGICAL CANCER, vol. 21, no. 2, 1 February 2011 (2011-02-01), pages 269-279, XP055283541, US ISSN: 1048-891X, DOI: 10.1097/IGC.0b013e31820e5cda

## Description

### Technical Field

The present invention relates to a method for identifying stem cells, which comprises analyzing an epigenetic change in chromosomal DNA extracted from stem cells, and making patterns from the obtained data.

### Background Art

As conventional methods for identifying stem cells, the following methods have been known, for example: (i) identification of a morphological change by observation under a microscope, (ii) a method of staining cells using antibodies for cell surface markers such as SSEA-3 or SSEA-4, and (iii) a method of staining cells with dye, utilizing enzyme activity possessed by the cells, such as alkaline phosphatase activity.

When pluripotent stem cells are passaged, it is frequently observed that the properties of the stem cells are changed, while no abnormal change is detected in terms of the morphological properties of the stem cells.

Examples of such a change in properties include (a) a change in differentiation tendency by differentiation induction operations, (b) a change in the cell growth rate, and (c) a change in resistance to canceration.

However, according to the aforementioned conventional identification methods, the changes in the properties of cells (a) to (c) cannot be identified.

As an example showing the limitation of the conventional identification methods, there is the report described in the International Stem Cell Initiative (Non Patent Literature 1). In the International Stem Cell Initiative, 59 types of human ES cells provided from 17 research institutes in various countries over the world have been used, and studies have been conducted regarding the expression of SSEA3, SSEA4, TRA-1-60, TRA-1-81, GCTM343, CD9, CD90, ALP. Class1 HLA, and the like that are antigens specific to stem cells, and the gene expression of NANOG, POU5F1(OCT4), TDGF1, DNMT3B, GDF3, and the like that are genes specific to stem cells (Non Patent Literature 1). As a result, it has been reported that all of the 59 types of human stem cells exhibited similar expression regarding these antigens and related genes, although there were several exceptions. From these results, it is found that a difference in the properties of stem cells among cell lines cannot be grasped by morphological observation.

Thus, as a next stage, an attempt has been made to grasp a change in cells at a genetic level.

As such an attempt, first, the expression analysis of mRNA and the single nucleotide polymorphism (SNP) analysis of chromosomal DNA have been carried out on various types of stem cells. A large number of stem cell lines were analyzed. However, no differences could be found among cell lines, in terms of gene expression pattern and SNP. A gene group causing the aforementioned changes in the properties of cells (a) to (c) has not yet been specified.

Next, as a means for fundamentally examining the mechanism of regulating gene expression, epigenetic analysis has been expected (Non Patent Literature 3).

As targets of such epigenetic analysis, in particular, methylation modification (5-methylcytosine (5mC)) of chromosomal DNA and modification of histone proteins have been focused. Since 1980s, it had been suggested that methylation of DNA be deeply associated with regulation of gene expression. For many years, through trial and error, the development of a methylation detection method has been intended, and from about the year 2000, a bisulfite method has been used as a detection method involving 5mC modification.

The bisulfite method is a method which comprises allowing chromosomal DNA to react with sodium sulfite (bisulfite) and converting an unmodified cytosine residue to uracil (bisulfite conversion). Moreover, a method of combining bisulfite conversion with allele-specific PCR or a restriction enzyme treatment has also been developed, and the analysis of individual genes has been promoted.

Furthermore, as improved methods thereof, an analysis method in which the bisulfite method is combined with a microarray (Non Patent Literature 2) and an analysis method in which the bisulfite method is combined with a next generation sequence method (Bis-seq method) (Non Patent Literature 4) have been developed. According to these methods, it has become possible to comprehensively analyze the state of the chromosomal DNA of stem cells.

In particular, the Bis-seq method makes it possible to analyze a methylation pattern in a one-to-one nucleotide basis. However, the Bis-seq method has a certain detection limit, regarding which it qualitatively detects the presence or absence of methylation modification but it cannot quantitatively detect the possibility of methylation. Moreover, the Bis-seq method is disadvantageous in that a single sample needs to be sequenced repeatedly 10 or more times, and thus, this method requires great care and is highly expensive. For these reasons, only a limited number of laboratories could conduct the Bis-seq method.

By the way, in recent years, it has been confirmed that cytosine modification includes not only 5mC but also 5-hydroxymethylcytosine (5hmC) (Non Patent Literature 2). Moreover, it has been reported that, as in the case of 5mC having resistance to bisulfite conversion (namely, 5mC is not converted to uracil by a bisulfite treatment), 5hmC is also resistant to bisulfite conversion (Non Patent Literatures 5 and 6).

According to this report, when bisulfite-treated DNA is subjected to sequence analysis, an unmodified cytosine is decoded as uracil (namely, thymine), whereas 5mC and 5hmC are each decoded as cytosine. That is, it has been revealed that 5mC cannot be distinguished from 5hmC according to the conventional Bis-seq analysis, and that both 5mC and 5hmC are present in the obtained data.

It has been pointed out that 5hmC plays an important role in the demethylation mechanism of chromosomal DNA, and thus, 5hmC is considered to be an intermediate in a reaction of converting a methylcytosine to an unmodified cytosine.

Since the Bis-seq method is unable to distinguish 5mC from 5hmC, this method cannot be considered to be an analysis method sufficient for tracing an epigenetic change in cells.

On the other hand, an MBD-seq method, which comprises concentrating a methylated DNA fragment using an MBD protein that recognizes 5mC, and then analyzing the obtained fragment using a next generation sequencer, has been developed (Non Patent Literature 7). However, in the case of the MBD-seq method, it has been known that since bimodal concentration peaks are obtained upon reading the concentrated DNA fragment, the estimated modification position is shifted by approximately 100 to 200 bps every time. In this method, candidate genes are narrowed by assuming a gene region located near a DNA region, in which methylation has been detected, as a regulatory gene. However, this method has a certain limit in that it cannot clearly specify the gene.

Furthermore, an MIRA method, which comprises analyzing a DNA fragment of 5mC that has been concentrated with an MBD protein by microarray, has also been developed (Non Patent Literature 8). This method is able to precisely concentrate only the 5mC fragment, and thus, it is considered that more exact data can be obtained by this method than by the Bis-seq method. However, the MIRA method cannot clearly identify subtle properties of stem cells, in that (i) as with the MBD-seq method, this method can merely select a gene located near a methylation region as a candidate gene and it cannot specify a gene regulated by methylation, and further, the possibility of methylation cannot be quantitatively detected, and in that (ii) this method has only 5mC as an analysis target and it cannot trace a change in 5hmC. WO2012/037456 discloses a method for selecting a pluripotent stem cell line, comprising measuring DNA methylation of a set of target genes in the pluripotent stem cell line, and performing a comparison of the DNA methylation data with a reference DNA methylation data of the same target genes. Techniques such as MeDIP-Chip are mentioned.

[Citation List]

[Non Patent Literature]

[Non Patent Literature 1] Nature Biotechnology, volume 25, No. 7, 803-816, 2007
[Non Patent Literature 2] James R. Tollervey and et al., Epigenetics 7, 823-840 (2012)
[Non Patent Literature 3] Koichiro Nishino, and et al., PLoS one 5, e13017 (2010)
[Non Patent Literature 4] Julia Arand and et al., PLoS one, 8, e1002750 (2012)
[Non Patent Literature 5] Colm Nestor and et al., BioTechnique 48, 317-319 (2010)
[Non Patent Literature 6] Yun Huang and et al., PLoS one 5, e8888 (2010)
[Non Patent Literature 7] Li N and et al., Methods, 52,203-212 (2010)
[Non Patent Literature 8] Joshua D. Tompkins and et al., PNAS, 109, 12544-12549 (2012)

Disclosure of Invention

Under the aforementioned circumstances, it is an object of the present invention to provide a method for identifying stem cells, as defined in the appended claims, which is used to precisely grasp the quality and/or properties thereof.

The present inventors have conducted the epigenetic analysis of stem cells, using SX-8G Compact, an apparatus developed by Precision System Science Co., Ltd. (hereinafter referred to as "PSS"). The inventors have simultaneously performed auto-methylated DNA immunoprecipitation (Auto MeDIP) and auto-hydroxymethylated DNA immunoprecipitation (Auto h-MeDIP) on chromosomal DNA samples extracted from embryonic stem cells (ES cells) and induced pluripotent stem cells (iPS cells) having different passage numbers, and have then conducted a microarray analysis on the obtained samples (MeDIP/h-MeDIP on chip).

The obtained microarray data were subjected to a numerical treatment using algorithms, and mapping was then carried out using UCSC Genome Browser, so that the methylation patterns of individual genes were analyzed.

From the obtained results, the present inventors have found that ES cells in the initial stage and late stage of passage, which could not be distinguished from each other by the conventional methods, can be clearly identified based on the methylation and hydroxylmethylation patterns of genes, thereby completing the present invention.

Present disclosure relates to the following:

[1] A method for identifying stem cells, which comprises analyzing the patterns of methylation and demethylation of chromosomal DNA extracted from test stem cells, and correlating the analyzed patterns with the properties of the test stem cells.

[2] The method according to [1] above, wherein the analysis of the patterns of methylation and demethylation is carried out by immunoprecipitation, a hybridization treatment using a microarray, a treatment of probability values of signal data obtained by the hybridization treatment, and the mapping of the probability values.

[3] The method according to [2] above, wherein the mapping of the probability values is carried out by assigning the methylation probability values $P_m$ and demethylation probability values $P_{hm}$ of individual probes on a microarray to probe numbers.

[4] The method according to [3] above, wherein the mapping of the probability values further comprises the following steps:

(1) a step of selecting the probe number n (wherein n is an integer) of a test stem cell and the microarray probe number n' (wherein n' is an integer) of a reference stem cell corresponding to the probe number n,

(2) a step of obtaining the ratio ($r = P_{(n)} / P_{(n')}$) of the probability value $P_{(n)}$ assigned to the probe number n to the probability value $P_{(n')}$ assigned to the probe number n',

(3) a step of repeating the step (1) from the continuous probe numbers n to n + i (wherein i is an integer) and then counting the number $S_r$ of the ratio r that is in the range of 0.5 to 1.5, and

(4) regarding each of the methylation probability values and the demethylation probability values, a step of obtaining the following correlation percentage:

$$R (\%) = \{S_r / (i + 1)\} \times 100.$$

[5] The method according to [1] above, wherein the properties of stem cells are used to characterize the stage of passage or differentiation.

[6] The method according to [1] above, wherein the analysis of the patterns of methylation and demethylation is carried out using automated equipment.

[7] The method according to [1] above, wherein the stem cells are ES cells or iPS cells.

[8] The method according to [1] above, wherein the stem cells are ectodermal, endodermal or mesodermal stem cells.

[9] The method according to [4] above, wherein the correlation of the analyzed patterns with the properties of the test stem cells is carried out based on the following criteria (a) to (d):

(a) when the methylation correlation percentage $R_m$ is 70% or more and the demethylation correlation percentage $R_{hm}$ is 70% or more, the test stem cells and the reference stem cells are in a similar state in terms of both methylation and demethylation,

(b) when the methylation correlation percentage $R_m$ is less than 70% and the demethylation correlation percentage $R_{hm}$ is 70% or more, the test stem cells and the reference stem cells are in a similar state in terms of demethylation, but are in a dissimilar state in terms of methylation,

(c) when the methylation correlation percentage $R_m$ is 70% or more and the demethylation correlation percentage $R_{hm}$ is less than 70%, the test stem cells and the reference stem cells are in a similar state in terms of methylation, but are in a dissimilar state in terms of demethylation, and

(d) when the methylation correlation percentage $R_m$ is less than 70% and the demethylation correlation percentage $R_{hm}$ is less than 70%, the test stem cells and the reference stem cells are in a dissimilar state in terms of both methylation and demethylation.

According to the present invention, it is possible to analyze the methylation and hydroxymethylation of various stem cells including iPS cells as typical examples, and to grasp or trace an epigenetic change in the stem cells.

That is to say, the present invention makes it possible to clearly distinguish the internal properties and states of cells, which cannot be distinguished based on antibody staining or gene expression, by epigenetic analysis. Not only a difference between undifferentiation and differentiation, but also two types of cell groups that are both in an undifferentiation state can be distinguished by the present invention, even though it is a small difference that cannot be distinguished based on antibody staining or gene expression.

Therefore, it is possible to select stem cells capable of being subjected to studies for drug discovery or medical treatments by identifying similarity or dissimilarity in epigenetic states among cell lines according to the method of the

present invention.

In addition, the method of the present invention can largely contribute to industrialization of the regenerative medicine field, such as the quality evaluation or quality control of stem cells, etc.

Brief Description of Drawings

Figure 1: Figure 1 is a schematic view showing a MeDIP method that is the method of the present invention.
Figure 2: Figure 2 is a view showing virtual data regarding methylation probability $P_m$ values.
Figure 3: Figure 3 is a view showing virtual data regarding hydroxymethylation probability $P_{hm}$ values.
Figure 4: Figure 4 is an electrophoretogram of chromosomal DNAs purified from stem cells.
Figure 5: Figure 5 is an electrophoretogram of DNA fragments concentrated by a MeDIP method.
Figure 6: Figure 6 is a mapping graph showing the methylation $P_m$ values of iPS cells.
Figure 7: Figure 7 is a mapping graph showing the methylation $P_m$ values of iPS cells.
Figure 8: Figure 8 is a mapping graph showing the methylation $P_m$ values of iPS cells.
Figure 9: Figure 9 is a mapping graph showing the methylation $P_m$ values of iPS cells.
Figure 10: Figure 10 is a mapping graph showing the methylation $P_m$ values and hydroxymethylation $P_{hm}$ values of ES cells.
Figure 11: Figure 11 is a mapping graph showing the methylation $P_m$ values and hydroxymethylation $P_{hm}$ values of ES cells.
Figure 12: Figure 12 is a mapping graph showing the methylation $P_m$ values and hydroxymethylation $P_{hm}$ values of ES cells.
Figure 13: Figure 13 is a mapping graph showing the methylation $P_m$ values and hydroxymethylation $P_{hm}$ values of ES cells.
Figure 14: Figure 14 is a mapping graph showing the methylation $P_m$ values of ES cells.
Figure 15: Figure 15 is a table summarizing numerical value data regarding the methylation $P_m$ values of ES cells.

Best Mode for Carrying Out Invention

Hereinafter, the present invention will be described in detail. The following embodiments are provided for illustrative purpose only for explaining the present invention, and thus, these embodiments are not intended to limit the scope of the present invention. The present invention can be carried out in various forms, unless it deviates from the gist thereof.

In addition, the present description makes reference to all of the contents as disclosed in the specification and drawings of Japanese Patent Application No. 2013-005594 filed on January 16, 2013, which is a priority document of the present application.

Terms

In the present application, with regard to terms such as the probability values P of methylation or hydroxymethylation, the ratio r of probability values P, correlation percentage R, and correlation coefficient R', in order to clearly distinguish whether these terms are relevant to either methylation or hydroxymethylation, there may be cases in which the symbol "m" indicating methylation or the symbol "hm" indicating hydroxymethylation is added as a subscript to the right of the terms. Otherwise, when it is contextually clear that the aforementioned terms are relevant to either methylation or hydroxymethylation, the aforementioned symbols may be omitted in some cases.

1. Method for identifying stem cells

The present disclosure provides a method for identifying stem cells, which enables a clear distinction between (among) two or more types of stem cell lines in terms of a difference in the properties of the stem cell lines by subjecting the chromosomal (genomic) DNAs of the two or more types of stem cell lines to epigenetic analysis.

Specifically, the present disclosure provides a method for identifying stem cells, which comprises analyzing the patterns of methylation and demethylation (which is also referred to as "hydroxymethylation") of chromosomal DNA extracted from test stem cells, and correlating the analyzed patterns with the properties of the test stem cells.

The process of differentiation and dedifferentiation (reprogramming) of stem cells is basically an epigenetic change that does not involve a change in the gene sequence, and among others, the methylation and demethylation of DNA are particularly changes that are cores of differentiation and dedifferentiation.

Methylation

DNA methylation occurs on cytosine among 4 types of nucleotides that constitute DNA, namely, adenine (A), guanine (G), thymine (T) and cytosine (C). In the methylated cytosine (methylcytosine), the carbon at position 5 on the pyridine ring thereof is substituted with a methyl group ($-CH_3$), as shown in the following formula.

[Formula 1]

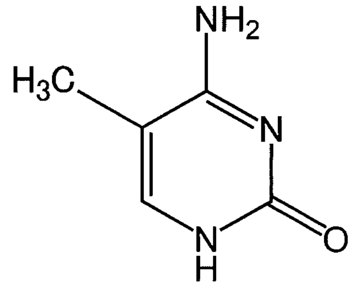

Demethylation

In chromosomal DNA, the methyl group of 5-methylcytosine is not always present, and it is lost by demethylation. As an intermediate during the demethylation process, 5-hydroxymethylcytosine is generated. In the 5-hydroxymethylcytosine, the carbon at position 5 on the pyridine ring thereof is substituted with a hydroxymethyl group ($-CH_2OH$) (see the following formula).

[Formula 2]

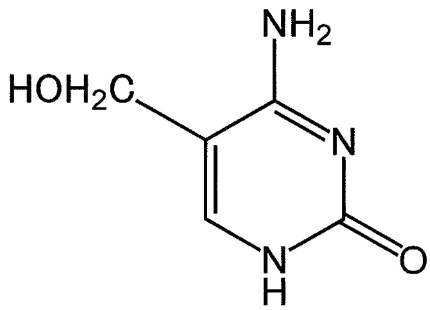

It has been known that the methylation state and demethylation state of DNA are always changed, and that the methylation and demethylation states of DNA are significantly changed, in particular, in stem cells.

Hence, in the identification method of the present invention, stem cells are used as targets.

The "stem cells" that are the targets of the present invention may be any of adult stem cells, embryonic stem (ES) cells, and induced pluripotent stem (iPS) cells.

Moreover, the stem cells may be derived from mammals. Examples of the mammal include a human, a monkey, a horse, a bovine, sheep, a goat, a swine, a dog, a rat and a mouse, but the examples are not limited thereto.

In one embodiment, the stem cells are derived from a human, whereas in another embodiment, the stem cells are derived from a non-human mammal such as a mouse.

Adult stem cells may be any of ectodermal, endodermal and mesodermal stem cells. Such adult stem cells may have or may not have pluripotency. Examples of the adult stem cells include neural stem cells, hematopoietic stem cells, mesenchymal stem cells, hepatic stem cells, pancreatic stem cells, skin stem cells, muscle stem cells, and germ stem cells.

ES cells are stem cells having pluripotency, which can be collected from the internal cell mass of embryo (Strelchenko N et al., (2004) Reprod Biomed Online. 9: 623-629.). In the present disclosure, ES cells are not limited to a primary cell line collected from such an internal cell mass, and they may be an ES cell line that has already been established as a cell line. Examples of the established ES cell line include a cell line distributed from a cell population

that has been obtained by allowing the already established ES cell line to proliferate, and an ES cell line obtained by melting the cryopreserved ES cell line and culturing it. If the ES cells used herein are such an established cell line, they can be acquired without passing through a step of disintegrating a fertilized egg.

Otherwise, ES cells may be established using only a single blastomere of embryo in the cleavage stage before the blastocyst stage, without impairing the developmental potency of the embryo. This is because these ES cells can be obtained without destroying a fertilized egg (Klimanskaya I et al., (2006) Nature 444: 481-485; and Chung Y et al., (2008) Cell Stem Cell 2: 113-117).

A method of culturing human-derived ES cells is described in RIKEN CDB, Human Stem Cell research Support Office Protocols (2008), Takahashi, K. et al. (Cell (2007), Nov 30: 131, pp. 861-872) and Thomson, J.A. et al., (Science (1998) Nov 6: 282, pp. 1145-1147).

iPS cells mean undifferentiated pluripotent stem cells obtained by introducing an initialization gene (a gene such as Oct3/4, Sox2, c-Myc, Klf4, NANOG, or LIN28) into the somatic cells (fibroblasts, epithelial cells, etc.) of mammals (including humans). The chromosomal DNA of iPS cells is reprogrammed by introduction of an initialization gene, and iPS cells basically have the same pluripotency as that of ES cells (Japanese Patent Laid-Open No. 2009-165478; Takahashi K et al., (2006) Cell 126: 663-676; and Takahashi K et al., (2007) Cell 131: 861-872).

iPS cells can be cultured as the same method as that applied to ES cells.

In undifferentiated cells such as ES cells and iPS cells, the methylation state of chromosomal DNA is particularly unstable. For example, if two or more iPS cell lines are present and even if all of the cell lines express an undifferentiation marker protein or gene, such as OCT3/4, SSEA4 and/or NANOG, the methylation state of the chromosomal DNA is different among the cell lines in many cases. In addition, it is considered that a difference in proliferative ability and differential ability (ability to differentiate into a specific lineage or tissue) is generated among the cell lines, depending on such a difference in the methylation state.

The present invention enables detection of a difference in the methylation state of chromosomal DNA, which cannot be detected only by the expression analysis of an undifferentiation marker. According to the method of the present invention, the methylation state of test stem cells is analyzed, and the analytical results are compared with the methylation state of reference stem cells having high undifferentiated state-maintaining ability, high proliferative ability or high differential ability, so that the properties of the test stem cells (undifferentiated state-maintaining ability, proliferative ability, differential ability, etc.) can be determined.

Analysis of methylation and hydroxymethylation patterns

First, chromosomal DNA is extracted from the above-described test stem cells. The cells are subjected to cell lysis according to an alkaline SDS method, a protease K digestion method, a chaotropic ion dissolution method, or the like, and thereafter, chromosomal DNA can be extracted from the cell lysate according to a silica bead method, a silica membrane method, an anion exchange resin column method, a DEAE kneading membrane method, a silica magnetic bead method, an -OH based magnetic bead method, an ion exchange magnetic bead method, or the like. The extracted chromosomal DNA may be further purified by an ethanol precipitation method or the like.

Next, the region on the obtained chromosomal DNA, which undergoes methylation and hydroxymethylation, is analyzed. Regarding the analysis of DNA methylation and hydroxymethylation, various methods have been established.

An example of the method of analyzing methylation is methylated DNA immunoprecipitation (MeDIP). Other examples include an MIAMI method (Microarray-based Integrated Analysis of Methylation by Isoschizomers) and a method using Infinium Human Methylation 450 BeadChip (Illumina).

On the other hand, an example of the method of analyzing hydroxymethylation is hydroxymethylated DNA immunoprecipitation (hMeDIP). Other examples include a method using Hydroxymethyl Collector or EpimarkTM 5-mC & 5-hmC Analysis Kit (New England Biolabs), an oxBS-Sequence method, and a TAB-Sequence method.

The method of analyzing methylation is preferably the MeDIP method, and the method of analyzing hydroxymethylation is preferably the hMeDIP method.

Hereinafter, the MeDIP method and the hMeDIP method will be given as examples, and these analysis methods will be specifically described (see Figure 1).

MeDIP method

(A) Fragmentation

The obtained chromosomal DNA is fragmented by ultrasonic disintegration or a treatment with suitable restriction enzymes (Figure 1: Step A). Thereby, a mixture comprising various chromosomal DNA fragments can be obtained. A portion of this mixture (Mixture 1) is collected as an input DNA sample, and the remaining mixture (Mixture 2) is subjected to immunoprecipitation using an anti-methylcytosine antibody. The input DNA sample means a sample to be hybridized

with a microarray probe competitively with a methylated DNA fragment that has been concentrated by the MeDIP method, when the methylated DNA fragment is hybridized with the microarray probe.

In the input DNA sample, both a methylated DNA fragment and an unmethylated DNA fragment are present.

By using the input DNA sample, the risk that the methylated DNA fragment concentrated by the MeDIP method non-specifically binds to the microarray probe, thereby generating pseudopositive results, can be reduced.

(B to D) Immunoprecipitation

A DNA fragment of the Mixture 2 is denatured to form a single stranded DNA fragment (Figure 1: Step B). The obtained single-stranded DNA fragment is allowed to react with an anti-methylcytosine antibody, and thereafter, a complex consisting of the antibody and the methylated DNA fragment is concentrated by centrifugation or magnetic separation and is then extracted (Figure 1: Step C).

The extracted complex consisting of the antibody and the methylated DNA fragment is treated with protease to remove the antibody or the DNA-binding protein therefrom, so that the methylated DNA fragment is purified (Figure 1: Step D).

(E) Fluorescent labeling of methylated DNA fragment

The purified methylated DNA fragment is amplified by PCR (Figure 1: Step E). A random probe is used in PCR amplification. At that time, a PCR product is labeled with a first fluorescent labeling (Cy5, etc.). Thereby, the fluorescently-labeled methylated DNA fragment (hereinafter referred to as a "MeDIP fragment") is obtained. In the example shown in Figure 1, the methylated DNA fragment is labeled with Cy5. However, the methylated DNA fragment may also be labeled with another suitable fluorescent labeling.

(F) Fluorescent labeling of input DNA

The input DNA obtained in the previous step "(A) Fragmentation" is treated with protease to purify the DNA fragment. The purified DNA fragment comprises both a methylated DNA fragment and an unmethylated DNA fragment. Subsequently, the generated input DNA is amplified by PCR. A random probe is used in PCR amplification. At that time, a PCR product is labeled with a second fluorescent labeling (a fluorescent labeling having an excitation wavelength and/or an emission wavelength different from those of the first fluorescent labeling) (Figure 1: Step F).

Thereby, a mixture of the methylated DNA fragment and the unmethylated DNA fragment, which has been labeled with the second fluorescent labeling, is obtained (hereinafter referred to as an "input fragment"). In the example shown in Figure 1, the input fragment is labeled with Cy3. However, the input fragment may also be labeled with another suitable fluorescent labeling.

(G) Microarray analysis

The obtained MeDIP fragment is analyzed by microarray. Since the position of a probe is fixed in the microarray, errors regarding positional information or differences in algorithms are hardly generated, and thus, a gene can be identified more precisely. Moreover, the microarray is much more inexpensive than sequence analysis methods such as a next generation sequence method. Moreover, since the data analysis can also be easily carried out in a short time in the microarray, this method is considered to be a more excellent method for identifying stem cells.

Furthermore, in the case of the microarray, since probes can be easily designed and modified, they can be designed, for example, such that DNA regions to be detected can be partially overlapped with one another (e.g., a tiling array, etc.), and thereby, methylation and hydroxymethylation sites can be specified in detail.

As specific procedures, first, a fluorescently-labeled MeDIP fragment and an input fragment are allowed to competitively react with microarray probes (Figure 1: Step G).

A Stanford-type microarray is preferably used.

Further, in the competitive reaction, the ratio between the MeDIP fragment and the input fragment is 1 : 2 to 1 : 50, preferably 1 : 5 to 1 : 30, and more preferably 1 : 7 to 1 : 20, 1 : 8 to 1 : 10, or 1 : 10.

As already described above, the input fragment can be obtained by PCR amplification using a methylated DNA fragment and an unmethylated DNA fragment as templates. In contrast, the MeDIP fragment can be obtained by PCR amplification using a concentrated methylated DNA fragment as a template. Accordingly, when the input fragment is compared with the MeDIP fragment in a state in which the total DNA amounts (or total DNA concentrations) are adjusted to be equal, the MeDIP fragment shows a higher concentration than the input fragment, in terms of a methylated DNA fragment.

Thus, when the input fragment and the MeDIP fragment are allowed to competitively react with the microarray

probes, (i) the input fragment binds to a probe corresponding to an unmethylated DNA region, and (ii) the MeDIP fragment preferentially binds to a probe corresponding to a methylated DNA region.

Therefore, in a probe region in which the fluorescence intensity of the input fragment is high, it is highly likely that the chromosomal DNA would not be methylated (in other words, it is unlikely that the chromosomal DNA would be methylated), whereas in a probe region in which the fluorescence intensity of the MeDIP fragment is high, it is highly likely that the chromosomal DNA would be methylated.

Fluorescence intensity can be analyzed using program such as ACME (R software GSEA (Gen Set Enrichment Analysis)), MEDME (R software GSEA (Gen Set Enrichment Analysis)), or Batman.

hMeDIP method

hMeDIP can be carried out by performing the above-described Steps (A) to (G), using an anti-hydroxymethylcytosine antibody instead of an anti-methylcytosine antibody.

In this case, in a probe region in which the fluorescence intensity of the input fragment is high, it is highly likely that the chromosomal DNA would not be hydroxymethylated (in other word, it is unlikely that the chromosomal DNA would be hydroxymethylated), whereas in a probe region in which the fluorescence intensity of the hMeDIP fragment is high, it is highly likely that the chromosomal DNA would be hydroxymethylated.

Probability processing of signal data

As described above, in a probe region in which the fluorescence intensity of the input fragment is high, it is unlikely that the chromosomal DNA would be methylated, whereas in a probe region in which the fluorescence intensity of the MeDIP fragment is high, it is highly likely that the chromosomal DNA would be methylated.

Hence, by comparing the fluorescence intensity of the input fragment with the fluorescence intensity of the MeDIP fragment, both of which are released from the probes, and then analyzing an intensity difference, the probability $P_m$ that the DNA region corresponding to the probe is methylated can be obtained.

Likewise, the probability $P_{hm}$ that the DNA region corresponding to the probe is hydroxylmethylated can also be obtained.

The probabilities $P_m$ and $P_{hm}$ can be obtained by various calculation methods, and can be preferably obtained by a $\chi^2$ test or a Bayesian test.

The $\chi^2$ test and the Bayesian test can be carried out using an algorithm for microarray analysis, ACME (Algorithm for Capturing Microarray Enrichment) (Scacheri et al., Methods Enzymol. 2006; 411: 270-82.) and Batman (Bayesian Tool for Methylation Analysis) (Rakyan VK et al. (2008) Genome Res 18: 1518-1529), respectively.

The above-described MeDIP analysis and hMeDIP analysis can be automated. Examples of automated equipment used in the MeDIP and hMeDIP analyses include SX-8G Compact (PSS), SX-8G (PSS), 6GC (PSS), 12GC (PSS), 12GC Plus (PCC), EZ1 (Qiagen), and Target Angler (TAMAGAWA SEIKI CO., LTD.), but the examples are not limited thereto.

In a preferred aspect, SX-8G Compact (PSS) is used herein as automated equipment.

SX-8G Compact is automated equipment that has been developed by the present inventors, as well as basic automatic protocols, software, and automatic reagents. Using this automated equipment, experimental operations for epigenetic immunoprecipitation (MeDIP, hMeDIP, ChIP, or the like) can be automated.

By automating the experimental operations, a variation in data which may be caused by manual works, false recognition caused by noise, and the like can be avoided. As a result, high reproducibility and reliability can be imparted to the data.

Mapping of probability values

By assigning the obtained methylation probability values $P_m$ to individual probe numbers on the microarray, the mapping of probability values $P_m$ is carried out (see Figure 2). The mapping can be carried out using a suitable browser such as UCSC Genome Browser. Other than this, the mapping can also be carried out using Integrated Genome Browser (IGB) (Nicol JW et al., Bioinformatics. 2009 Aug 4 [1]; Helt GA et al., BMC Bioinformatics. 2009 Aug 25; 10(1): 266. [2]), Signal Map (NimbleGen), Genomic WorkBench (Agilent), or the like.

Figure 2 is a schematic view showing a mapping graph that has been obtained when the mapping is carried out by setting the longitudinal axis as P value and setting the horizontal axis as probe number (graph A: the $P_m$ values of the test stem cells; and graph B: the $P_m$ values of the reference stem cells). The probe number at the horizontal axis corresponds to the position on the chromosome. The data shown in Figure 2 are virtual data. Hereinafter, referring to this virtual data as an example, the step of treating data of the present invention will be described.

By mapping, the methylation probability values $P_m$ correspond to probe numbers. Hence, concerning the corresponding probe numbers, as described below, the ratio between the $P_m$ values of test stem cells and the $P_m$ values of reference stem cells can be obtained. Then, concerning a methylation pattern, the correlation percentage R between the test stem cells and the reference stem cells can be obtained.

In the present invention, the probe number n (wherein n represents a natural number) of the microarray used in the analysis of the test stem cells corresponds to (is matched with) the probe number n' (wherein n' represents a natural number) of the microarray used in the analysis of the reference stem cells.

The ratio $r_{(n)} = P_{m(n)}/ P_{m(n')}$ is obtained from the methylation probability $P_{m(n)}$ of the probe number n of the test stem cells and the methylation probability $P_{m(n')}$ of the corresponding probe number n' of the reference stem cells. Likewise, regarding the probe numbers n + 1, n + 2, ... n + i (wherein i represents an integer) (in the case of the reference stem cells, regarding n' + 1, n' + 2, ... n' + i), the ratios $r_{(n+1)}$, $r_{(n+2)}$, ... $r_{(n+i)}$ are obtained.

Herein, individual probes with the numbers n to n + i (and n' to n' + i) correspond to continuous regions of chromosomal DNA (for example, one type of promoter region, etc.).

As an example, graph A shows the methylation probabilities $P_{m(n)}$ to $P_{m(n+3)}$ of the probe numbers n to n + 3. Likewise, graph B shows the methylation probabilities $P_{m(n')}$ to $P_{m(n'+3)}$ of the probe numbers n' to n' + 3.

In the case of the example shown in Figure 2, $P_{m(n)} = 1.0$, $P_{m(n+1)} = 0.5$, $P_{m(n+2)} = 0.8$, and $P_{m(n+3)} = 0.4$ for the test stem cells, whereas $P_{m(n')} = 0.9$, $P_{m(n'+1)} = 0.25$, $P_{m(n'+2)} = 0.6$, and $P_{m(n'+3)} = 1.0$ for the reference stem cells.

In this example, the ratio $r_{(n)} = P_{m(n)}/ P_{m(n')} = 1.0/0.9 = 1.11$, and likewise, the ratio $r_{(n+1)}= 0.5/0.25 = 2$, $r_{(n+2)}= 0.8/0.6 = 1.33$, and $r_{(n+3)}= 0.4/1.0 = 0.4$.

Among the obtained $r_{(n)}$, $r_{(n+1)}$ ... $r_{(n+1)}$, the number $S_r$ of the ratio r in which the value is in a certain range is counted. In a certain aspect, the numerical value range of the ratio r that is counted as $S_r$ is $0.5 \leq r \leq 1.5$, and in another aspect, it is $0.6 \leq r \leq 1.4$, $0.7 \leq r \leq 1.3$, $0.8 \leq r \leq 1.2$, or $0.9 \leq r \leq 1.1$.

From the obtained $S_r$, the correlation percentage R (%) = $\{S_r / (i + 1)\} \times 100$ is obtained.

In the case of the example shown in Figure 2, the ratio $r_{(n)} = 1.11$, $r_{(n+1)}= 2$, $r_{(n +2)}= 1.33$, and $r_{(n+3)}= 0.4$. The values included in the range of $0.5 \leq r \leq 1.5$ are $r_{(n)} = 1.11$ and $r_{(n+2)}= 1.33$. Accordingly, in this case, $S_r = 2$.

Thus, if the correlation percentage R (%) is obtained,

$$R\ (\%) = \{S_r / (i + 1)\} \times 100 = \{2 / (3 + 1)\} \times 100 = 50\%.$$

Likewise, in the case of hydroxymethylation as well, with regard to the $P_{hm}$ value at any given probe number, the ratio r, the number $S_r$ of the ratio r in which the value is in a certain range, and the correlation percentage R (%) can be obtained.

Figure 3 shows the example (graph C: the $P_{hm}$ values of the test stem cells; and graph D: the $P_{hm}$ values of the reference stem cells).

In the case of the example shown in Figure 3, $P_{hm(n)} = 0.8$, $P_{hm(n+1)} = 0.5$, $P_{hm(n+ 2)} = 0.4$, and $P_{hm(n+3)} = 0.9$ for the test stem cells, whereas $P_{hm(n')} = 0.9$, $P_{hm(n'+1)} = 0.6$, $P_{hm(n' +2)} = 0.3$, and $P_{hm(n'+3)} = 1.0$ for the reference stem cells.

In this example, the ratio $r_{(n)} = P_{hm(n)}/ P_{hm(n')} = 0.8/0.9 = 0.99$, and likewise, $r_{(n+ 1)} = 0.5/0.6 = 0.83$, $r_{(n+2)} = 0.4/0.3 = 1.33$, and $r_{(n+3)} = 0.9/1.0 = 0.9$.

The values included in the range of $0.5 \leq r \leq 1.5$ are $r_{(n)} = 0.99$, $r_{(n+1)} = 0.83$, $r_{(n +2)} = 1.33$, and $r_{(n+3)} = 0.9$. Accordingly, in this case, $S_r = 4$.

Thus, if the correlation percentage R (%) is obtained,

$$R\ (\%) = \{S_r / (3 + 1)\} \times 100 = \{4 / (3 + 1)\} \times 100 = 100\%.$$

Hereinafter, the correlation percentages of methylation and hydroxymethylation are referred to as $R_m$ and $R_{hm}$, respectively.

Correlation of analyzed patterns with properties

The numerical values of the thus obtained correlation percentages R (%) can be correlated with the properties (in particular, the epigenetic state) of test stem cells according to the following criteria.

That is to say, the following criteria are applied.

(a) When the methylation correlation percentage $R_m$ is a value that is greater than a reference value X, and the hydroxymethylation correlation percentage $R_{hm}$ is a value that is greater than the reference value X, it is determined that the test stem cells and the reference stem cells are in a similar state in terms of both methylation and demethylation.

(b) When the methylation correlation percentage $R_m$ is a value that is less than a reference value X, and the hydroxymethylation correlation percentage $R_{(hm)}$ is a value that is greater than the reference value X, it is determined that the test stem cells and the reference stem cells are in a similar state in terms of hydroxymethylation, but are in a dissimilar state in terms of methylation.

(c) When the methylation correlation percentage $R_m$ is a value that is greater than a reference value X, and the hydroxymethylation correlation percentage $R_{hm}$ is a value that is less than the reference value X, it is determined that the test stem cells and the reference stem cells are in a similar state in terms of methylation, but are in a dissimilar state in terms of hydroxymethylation.

(d) When the methylation correlation percentage $R_m$ is a value that is less than a reference value X, and the hydroxymethylation correlation percentage $R_{hm}$ is a value that is less than the reference value X, it is determined that the test stem cells and the reference stem cells are in a dissimilar state in terms of both methylation and hydroxymethylation.

In the above-described criteria (a) to (d), the "reference value X" can be selected, as appropriate, depending on the types of test stem cells and reference stem cells, differentiation stage, and passage stage. In an embodiment, the reference value X is set at 60%, and it is set preferably at 70%, and more preferably at 80%, 90% or 95%.

In the example of the virtual data shown in Figure 2 and Figure 3, $R_m$ = 50%, and $R_{hm}$ = 100%.

When the reference value X is set at 60%, the virtual data shown in Figure 2 and Figure 3 correspond to the aforementioned case "(b) the methylation correlation percentage $R_m$ is less than 60%, and the hydroxymethylation correlation percentage $R_{hm}$ is 60% or more". Accordingly, it is determined that the test stem cells and the reference stem cells are in a similar state in terms of hydroxymethylation, but are in a dissimilar state in terms of methylation.

Aspect of using correlation coefficient R'

In a second aspect, the waveform of a mapping graph of P values is compared between the test stem cells and the reference stem cells, so that the correlation coefficient R' can be obtained. The correlation coefficient R' can be obtained using commercially available software and the like, such as free software such as R, SAS (SAS), SPSS (SPSS), Stat (Informatics), StatView (SAS), STATISTICA (StatSoft), SigmaStat (HULINKS), SYSTAT (HULINKS), MINITAB (Informatics), Prism (MDF), JMP (SAS), and Excel (Microsoft).

In this case, the numerical value of the correlation coefficient R' can be correlated with the properties of the test stem cells according to the following criteria (a') to (d').

That is to say, the following criteria are applied.

(a') When the methylation correlation coefficient $R'_m$ satisfies the reference value $X' \leq R'_m \leq 1.0$ (wherein the reference value X' is $0 < X' < 1$), and the hydroxymethylation correlation coefficient $R'_{hm}$ satisfies the reference value $X' \leq R'_{hm} \leq 1.0$, it is determined that the test stem cells and the reference stem cells are in a similar state in terms of both methylation and hydroxymethylation.

(b') When the methylation correlation coefficient $R'_m$ satisfies $R'_m <$ the reference value X', and the hydroxymethylation correlation coefficient $R'_{hm}$ satisfies the reference value $X' \leq R'_{hm} \leq 1.0$, it is determined that the test stem cells and the reference stem cells are in a similar state in terms of hydroxymethylation, but are in a dissimilar state in terms of methylation.

(c') When the methylation correlation coefficient $R'_m$ satisfies the reference value $X' \leq R'_m \leq 1.0$, and the hydroxymethylation correlation coefficient $R'_{hm}$ satisfies $R'_{hm} <$ the reference value X', it is determined that the test stem cells and the reference stem cells are in a similar state in terms of methylation, but are in a dissimilar state in terms of hydroxymethylation.

(d') When the methylation correlation coefficient $R'_m$ satisfies $R'_m <$ the reference value X', and the hydroxymethylation correlation coefficient $R'_{hm}$ satisfies $R'_{hm} <$ the reference value X', it is determined that the test stem cells and the reference stem cells are in a dissimilar state in terms of both methylation and hydroxymethylation.

In the above-described criteria (a') to (d'), the "reference value X'" can be selected, as appropriate, depending on the types of test stem cells and reference stem cells, differentiation stage, and passage stage. In an embodiment, the reference value X' is set at 0.7, and it is set preferably at 0.8, and more preferably at 0.85, 0.9, or 0.95.

The correlation percentage R (%) (or the correlation coefficient R') can be obtained regarding any given chromosomal DNA region. For example, the correlation percentage R (%) (or the correlation coefficient R') may be obtained regarding the promoter region of a single gene, or the correlation percentage R (%) (or the correlation coefficient R') may also be obtained regarding a repeat region that does not contain a gene region including a plurality of genes, or genes (a microsatellite region, etc.).

In the present invention, as described above, a methylation state and a hydroxymethylation state are determined based on probability values. Accordingly, the probability values of methylation and hydroxymethylation are preferably accurate values.

Thus, in the method of the present invention, the analysis of methylation and hydroxymethylation patterns is carried out by automation using automated equipment.

If the pattern analysis is carried out by automation, experimental errors that may be caused by manual works (errors generated in solution amount, reaction time, stirring conditions, and the like) can be avoided.

The automated equipment is as already described above.

As described above, if similarity or dissimilarity in the methylation and hydroxymethylation is determined between the test stem cells and the reference stem cells, the cytological characteristics of the test stem cells can be identified based on the determination. Examples of the cytological characteristics that can be identified by the method of the present invention include a passage stage and a differentiation stage.

(1) Identification of passage stage of iPS cells

It is assumed that an iPS cell line capable of maintaining an undifferentiated state and proliferative ability that is favorable as reference stem cells would be used, and that iPS cell lines (in which the expression of an undifferentiation marker gene becomes positive) obtained by culturing the above-described reference stem cells and/or allowing the reference stem cells to grow, and then subjecting the resulting cells to multiple passages would be used as test stem cells 1 and 2.

Moreover, the test stem cells and the reference stem cells are analyzed in terms of the promoter region of an undifferentiation marker gene (Oct3/4, SSEA4, Nanog or the like), and the following assumption is obtained. That is, it is assumed: that it is determined that the test stem cells 1 are similar to the reference stem cells in terms of the hydroxymethylation state of the aforementioned promoter region, and are dissimilar to the reference stem cells in terms of the methylation state of the promoter region, and it is also determined that the test stem cells 2 are similar to the reference stem cells in terms of both a methylation state and a hydroxymethylation state.

In this case, the methylation probability values $P_m$ are compared between the test stem cells 1 and the reference stem cells. When the test stem cells 1 entirely exhibit higher $P_m$ values, an undifferentiation marker gene us tentatively expressed in the test stem cells. However, it can be determined that the promoter region thereof gradually undergoes methylation modification. Accordingly, it can be determined that, in the test stem cells 1, an undifferentiation marker gene enters an off-state and the undifferentiated state cannot be maintained in the future.

In contrast to this, the test stem cells 2 are similar to the reference stem cells in terms of both the methylation state and the hydroxymethylation state, and thus, it can be determined that the test stem cells 2 will tend to maintain a good undifferentiated state and high proliferative ability even in the future.

(2) Identification of differentiation stage into neural stem cells

A neural stem cell line having high proliferative ability as reference stem cells and high ability to differentiate into nerve cells is used. It is assumed that test stem cells 1 and 2 would be cell lines in a process of inducing differentiation of iPS cells into neural stem cells, and that at the present moment, the expression of a neural stem cell marker gene (Nestin, NCAM, etc.) has not yet been detected.

Moreover, the test stem cells and the reference stem cells are analyzed in terms of the promoter region of a neural stem cell marker gene (Nestin, NCAM, etc.). It is assumed that the test stem cells 1 would be similar to the reference stem cells in terms of the hydroxymethylation state of the promoter region but would be dissimilar to the reference stem cells in terms of the methylation state of the promoter region, and that the test stem cells 2 would be similar to the reference stem cells in terms of the methylation state of the promoter region but would be dissimilar to the reference stem cells in terms of the hydroxymethylation state of the promoter region.

In this case, the test stem cells 1 are compared with the reference stem cells in terms of the methylation probability value $P_m$. When the test stem cells 1 entire exhibit higher $P_m$ values, it is found that, at present, the expression of the neural stem cell marker gene is suppressed in the test stem cells 1. Moreover, since the test stem cells 1 are similar to the reference stem cells in terms of the hydroxymethylation state, it cannot be said that the test stem cells 1 have a high tendency of demethylation, in comparison to the reference stem cells. Accordingly, it can be said that there are no signs for demethylation of methylcytosine in the promoter region in the test stem cells 1.

Accordingly, it can be determined that the test stem cells 1 are at an initial stage of differentiation upon induction of the differentiation of iPS cells into neural stem cells.

On the other hand, since the test stem cells 2 are similar to the reference stem cells in terms of the methylation state, it is found that the expression of the neural stem cell marker gene is not strongly inhibited. In addition, the two types of cells are dissimilar to each other in terms of the hydroxymethylation state. Thus, when the test stem cells 2 are

compared with the reference stem cells in terms of the hydroxymethylation probability values $P_{hm}$, if the test stem cells 2 entirely exhibit higher $P_{hm}$ values, it is found that, at present, the expression of the neural stem cell marker gene is turned to "switch on" in the test stem cells 2.

Therefore, it can be determined that the test stem cells 2 are at a late stage of differentiation upon induction of the differentiation of iPS cells into neural stem cells.

Hereinafter, the present invention will be described in detail using the following Examples. However, the embodiments described in the examples are not intended to limit the scope of the present invention.

Examples

[Experimental Procedures]

Methylation/hydroxylmethylation analysis of stem cells by simultaneous comparison of methylated DNA immunoprecipitation (MeDIP) and hydroxylmethylated DNA immunoprecipitation (h-MeDIP) (MeDIP/h-MeDIP on Chip)

1. Preparation of chromosomal DNA and measurement of concentration and size thereof

Cell pellets of human ES cells skhES-1 and khES-3 indicated for reference only, (Kyoto University) and human iPS cell pellets (obtained from ReproCELL Incorporated), which had been preserved at a centigrade temperature of -80°C, were thawed, and the cells were then suspended in 100 to 200 uL of a 0.9% NaCl solution, so that the cell concentration was set at 1 x $10^6$ cells/100 μL.

The aforementioned solution (100 μL) was dispensed into a 1.5-mL tube, and it was then placed in an automatic nucleic acid purification device 12GC (manufactured by Precision System Science Co., Ltd.). 1 μL of 10 μg/mL Rnase was added to a chromosomal DNA purification reagent Well 10, so as to produce a reagent for preparation.

Moreover, as a protocol for preparation of chromosomal DNA, MagDEA DNA 20012GC v3 with Rnase ver 0.1 (manufactured by Precision System Science Co., Ltd.) was used. Approximately 40 minutes later, 20 to 50 μL of a chromosomal DNA solution was obtained (wherein the eluted amount is different depending on the eluted volume determined).

2 μL of the chromosomal DNA solution was placed on a small spectrophotometer Nanodrop, and the concentration and A260/A280 were then measured. As a result, 20 to 50 μL of chromosomal DNA having a concentration of 60 to 120 μg/μL and A260/A280 of about 1.8 to 1.9 was obtained.

Subsequently, 1% agarose electrophoresis was carried out in a 1 x TAE buffer system. As markers, Wide range marker (manufactured by TAKARA) and 2 μL of Lamda/HindIII (manufactured by Takara Bio, Inc.) were used. With regard to size, the chromosomal DNA was found to be a single band of approximately 20 Kbps (Figure 4: lane A).

2. Fragmentation of chromosomal DNA by ultrasonic disintegration

Appropriate amounts of 2 x TE and DNase/RNase Free water were added to the purified DNA solution, so as to prepare a 50 ng/μl chromosomal DNA solution dissolved in 1 x TE. 200 μL of the 50 ng/μl chromosomal DNA was added to a 1.5-mL tube, and it was then disintegrated under the following conditions. An ultrasonic disintegration device BioRuptor UCD-250 (manufactured by Tosho Denki K. K.) was cooled, and the chromosomal DNA was then disintegrated in a cooling water of 4°C at an output medium of 15 sec ON/15 sec OFF. The size of the disintegrated chromosomal DNA was measured by 1% agarose electrophoresis. As a result, it was confirmed that approximately 200 to 800 bps of the disintegrated DNA fragment was obtained (Figure 4: lane B).

3A. Methylated DNA immunoprecipitation (MeDIP)

Using an automatic epigenetics system (manufactured by Precision System Science Co., Ltd.), automatic immunoprecipitation was carried out. As a reagent, Auto MeDIP Kit reagent (manufactured by Diagenode) was used.

100 μL of Lysis Buffer of Auto MagDEA-IP kit (manufactured by Precision System Science Co., Ltd.) was added to a well. 1 μg of the chromosomal DNA that had been disintegrated in Step 2 was heated at 95°C for 3 minutes, and it was then quenched with ice water, so that the chromosomal DNA was denatured to single-stranded DNA. Thereafter, 1 μg of an anti-5-methylcytosine antibody (manufactured by Diagenode) was added to the denatured DNA solution, and automatic immunoprecipitation was then carried out using SX-8G System. Using a ChIP Type B protocol, the solution was stirred in a state in which the anti-5-methylcytosine antibody, magnetic beads, and the disintegrated chromosomal DNA coexisted. In order to eliminate foreign substances, an immune complex of the magnetic beads, the antibody and the DNA was automatically washed with two types of washing solutions four times.

3B. Hydroxylmethylated DNA immunoprecipitation (h-MeDIP)

Employing the same device as used in Step 3A, and using Auto h-MeDIP Kit (manufactured by Diagenode) as a reagent and Lysis Buffer of Auto MagDEA-IP kit, automatic immunoprecipitation was carried out. 1 μg of the chromosomal DNA that had been disintegrated in Step 2 was heated at 95°C for 10 minutes, and it was then quenched with ice water, so as to form single-stranded DNA. Thereafter, 1 μg of an anti-5-hydroxymethylcytosine antibody was added to the denatured DNA solution, and automatic immunoprecipitation was then carried out according to an automation system. Using a ChIP Type A protocol (indirect immunoprecipitation method), a complex of the anti-5-hydroxymethylcytosine antibody and the chromosomal DNA was formed, and the immune complex of the antibody and the DNA was then complemented with magnetic beads. In order to eliminate foreign substances, an immune complex of the magnetic beads, the antibody and the DNA was automatically washed with two types of washing solutions four times.

4. Protease K treatment

To the magnetic bead suspension (immunoprecipitation concentrated sample) after completion of the immunoprecipitation, 1 μL of a protease K solution (manufactured by Diagenode) was added. At the same time, 10 μL of a sample before the immunoprecipitation was added to 90 μL of Lysis Buffer to prepare an input sample. 1 μL of protease K was also added to the input sample. Subsequently, the immunoprecipitation concentrated sample and the input sample were each covered with a lid, and were then heated at 58°C for 15 minutes, so that the protein in the above-described complex was digested. Then, in order to inactivate the enzyme activity of the protease K, each sample was heated at 95°C for 15 minutes. After completion of the heating, the samples were centrifuged (1000 rpm, 25°C, 1 minute), and water droplets attached to each lid were then recovered.

5. Automatic purification of DNA fragment and measurement of concentration thereof

The solution obtained in Step 4 was equipped into an automatic epigenetic apparatus SX-8G, so that the DNA fragment was purified. As a reagent, Auto MagDEA-IP Kit was used, and as a purification protocol, MagPurification8_v2.HDL or MagPurification16_v2-1.HDL was used. As purified DNA, 10 to 13 μL of the DNA solution was recovered.

Subsequently, 2 μL of the chromosomal DNA solution was placed on a small spectrophotometer Nanodrop, and the concentration and A260/A280 were then measured. The concentration was found to be 1 to 2 ng/μl.

6. Amplification and purification of concentrated DNA fragment

Using WGA2 kit (manufactured by Sigma), 5-10 ng of the concentrated DNA fragment was amplified. After Fragmentation Buffer had been added to the DNA fragment, Library Preparation Buffer and Enzyme were added thereto without heat denaturation, and thereafter, a universal linker was added thereto.

Subsequently, using Wizard SV Gel and PCR Clean-Up System (manufactured by Promega), universal primers remaining in the WGA2 kit were eliminated. Thereafter, 1% agarose electrophoresis was carried out in a 1 x TAE buffer solution system. As markers, Wide range maker (manufactured by Takara Bio, Inc.) and 2 μL of Lamda/HindIII (manufactured by Takara Bio, Inc.) were used. 6 μL of water and 2 μL of 5 x Dye were added to 2 μL of the DNA sample, and the obtained mixture was then electrophoresed at 100 V for 25 min. It was confirmed that the WGA2 amplification fragment was amplified in a region of 500-2000 bps (Figure 5).

7. Microarray analysis

The DNA sample and the input sample, which had been concentrated by immunoprecipitation, were each labeled with the fluorochromes Cy5 and Cy3. After completion of the labeling, competitive hybridization was carried out at a concentration ratio of 1 : 10 on microarrays (manufactured by Agilent and Nimblegen). After completion of the hybridization for 16 hours, the resultant was washed with a washing solution, and signals were then detected by a fluorescence scanner. After completion of pigment correction, signal intensity was preserved as character data.

8. Numerical value processing of signal data

For calculation of P values in a methylated region, Batman algorithm (produced by Thomas A. Down) and ACME algorithm (produced by Sean Davis) were used. For the microarray manufactured by Agilent, a Batman program in the Methylation Module of analysis software Genomomic Work Bench (manufactured by Agilent) was used. On the other hand, for the microarray manufactured by Nimblegen, an ACME program was used.

9. Mapping of individual P values on UCSC Genome Browser

The P values were mapped in individual probe positions on the UCSC Genome Browser. The stem cells (line khES 1) on individual stages with different passages, namely, the cells (E1) in the initial stage of passage, the cells (E2) in the late stage of passage, and the differentiated cells (E3) obtained by differentiation of the E1 cells with retinoic acid, were mapped, while comparing the P values by MeDIP with those by hMeDIP. The results of MeDIP were used as an indicator for the methylation by 5-methylcytosine, and the results of hMeDIP were used as an indicator for the demethylation by 5-hydroxycytosine.

10. Analysis of methylation/hydroxymethylation patterns

According to the following procedures, the methylation and hydroxymethylation patterns of cytosine residues were analyzed.

(1) With regard to 5 probes located close to one another (which were defined as a single "probe group"), the ratio r of P values = line 1/line2 was obtained.
(2) The ratio r whose value is included in 1.3 - 0.7 was selected, and the number $S_r$ of the ratios r was counted.
(3) The correlation percentage R (%) = ($S_r$ / 5) x 100 was calculated, and the region of 80% to 100% was determined to be a similar pattern.

[Example 1]

Analysis of methylation/hydroxymethylation patterns of iPS cells

The above-described Steps 1 to 10 were performed on human iPS cells (hereinafter referred to as "hiPS cells"). As a result, the mapping graphs shown in Figures 6 to 9 were obtained.

In each of the mapping graphs shown in Figures 6 to 9, the upper graph shows the pattern of methylation P values, and the lower graph shows the pattern of unmethylation P values. In each of Figures 6 to 9, the upper panel and lower panel graphs show the patterns in the same DNA regions in the hiPS cell lines 1 and 2, respectively. In addition, Figures 6 to 9 show the patterns of different DNA regions.

The numerical value data of the patterns in methylation P values on the mapping graphs shown in Figures 6 to 9 are shown in Tables 1 to 4, respectively.

Table 1

Table 1: Numerical value data of methylation patterns of hiPS cells

| P value (set1) | P value (set5) | Ratio r | $S_r$ | Correlation percentage R (%) | Correlation coefficient R' |
|---|---|---|---|---|---|
| 1.072273016 | 0.925485611 | 0.863 | 2 | 40 | 0.920 |
| 1.032350421 | 0.363731474 | 0.352 | 2 | 40 | |
| 1.245631218 | 0.553581953 | 0.444 | 3 | 60 | |
| 0.904466987 | 0.340658307 | 0.377 | 4 | **80** | |
| 0.960355639 | 0.719685674 | 0.749 | 5 | **100** | |
| 0.826369703 | 0.736362278 | 0.891 | 4 | **80** | |
| 1.782044291 | 1.382513404 | 0.776 | 3 | 60 | |
| 1.467092395 | 1.214730024 | 0.828 | 2 | 40 | |
| 0.929525495 | 0.695897281 | 0.749 | 2 | 40 | |
| 0.077342309 | 0.159082144 | 2.057 | - | - | |
| 0.467960268 | 0.322441608 | 0.689 | - | - | |
| 1.953680634 | 1.34842515 | 0.690 | - | - | |
| 2.344535351 | 1.868728876 | 0.797 | - | - | |

Table 2

Table 2: Numerical value data of methylation patterns of hiPS cells

| P value (set1) | P value (set5) | Ratio r | $S_r$ | Correlation percentage R (%) | Correlation coefficient R' |
|---|---|---|---|---|---|
| 0.336295664 | 0.070129722 | 0.209 | 1 | 20 | 0.912 |
| 0.207465261 | -0.075320043 | -0.363 | 1 | 20 | |
| 0.754212618 | 0.26706332 | 0.354 | 2 | 40 | |
| 1.935695648 | 1.567573309 | 0.810 | 3 | 60 | |
| 0.425325066 | -0.033495512 | -0.079 | 2 | 40 | |
| 0.570759356 | 0.793000996 | 1.389 | 2 | 40 | |
| 1.264191747 | 1.188448191 | 0.940 | 2 | 40 | |
| 1.42162478 | 1.573449016 | 1.107 | 1 | 20 | |
| 0.731782138 | -0.024577551 | -0.034 | 0 | 0 | |
| -0.300696373 | 0.19048582 | -0.633 | 1 | 20 | |
| -0.167050198 | -0.468576461 | 2.805 | 2 | 40 | |
| 0.403710335 | 0.706794977 | 1.751 | 3 | 60 | |
| 0.895414531 | 0.057170022 | 0.064 | 4 | 80 | |
| 1.645899773 | 1.776567101 | 1.079 | 5 | 100 | |
| 1.174440145 | 1.465094686 | 1.247 | 5 | 100 | |
| 1.029984474 | 0.858399868 | 0.833 | 5 | 100 | |
| 0.666302264 | 0.596626282 | 0.895 | 5 | 100 | |
| 0.926390767 | 0.963873744 | 1.040 | 4 | 80 | |
| 2.532273054 | 2.620643616 | 1.035 | 3 | 60 | |
| 1.689413071 | 1.378983855 | 0.816 | 2 | 40 | |
| 1.521535754 | 1.477265954 | 0.971 | 1 | 20 | |
| 2.26559782 | 3.079804659 | 1.359 | 1 | 20 | |
| 0.439749688 | 0.150853932 | 0.343 | 1 | 20 | |
| 1.365247011 | 0.687813997 | 0.504 | 2 | 40 | |
| -0.943828642 | -0.552829921 | 0.586 | 3 | 60 | |
| 0.748033524 | 0.74979347 | 1.002 | 4 | 80 | |
| 0.804844558 | 0.492513359 | 0.612 | 3 | 60 | |
| 0.478041112 | 0.435259551 | 0.911 | 3 | 60 | |
| 1.101124883 | 1.121453524 | 1.018 | 3 | 60 | |
| 1.380548954 | 1.383467197 | 1.002 | 2 | 40 | |
| 0.782859921 | 0.503360987 | 0.643 | 2 | 40 | |
| -1.60187459 | -2.330072403 | 1.455 | 2 | 40 | |
| 0.362611353 | 0.441885293 | 1.219 | 3 | 60 | |
| -0.359005809 | 0.001898385 | -0.005 | - | - | |
| 0.350418299 | 0.422477394 | 1.206 | - | - | |
| 0.468025804 | -0.557252824 | -1.191 | - | - | |
| 1.057294488 | 0.831465304 | 0.786 | - | - | |

Table 3

Table 3: Numerical value data of methylation patterns of hiPS cells

| P value (set1) | P value (set5) | Ratio r | $S_r$ | Correlation percentage R (%) | Correlation coefficient R' |
|---|---|---|---|---|---|
| 1.018406153 | 0.783500135 | 0.769 | 1 | 20 | 0.701 |
| 0.535746872 | 0.335283369 | 0.626 | 0 | 0 | |
| 0.405411571 | -1.617402077 | -3.990 | 0 | 0 | |
| -0.436292112 | -1.768951416 | 4.055 | 0 | 0 | |
| 0.288967937 | -0.801520824 | -2.774 | 0 | 0 | |
| 0.759627938 | 0.482367456 | 0.635 | 0 | 0 | |
| 1.510571957 | 1.056686163 | 0.700 | 0 | 0 | |
| 0.573392987 | 0.815347791 | 1.422 | 0 | 0 | |
| 0.039606806 | -0.829012215 | -20.931 | 0 | 0 | |
| 0.239608154 | 0.393559992 | 1.643 | 0 | 0 | |
| -0.397005945 | -1.279105663 | 3.222 | 0 | 0 | |
| 0.488900661 | -0.182890102 | -0.374 | 0 | 0 | |
| 0.068058334 | -1.240397811 | -18.226 | 0 | 0 | |
| 0.392202705 | -0.042175036 | -0.108 | 0 | 0 | |
| 1.622750163 | 0.92629534 | 0.571 | 0 | 0 | |
| -0.073593125 | -1.438990235 | 19.553 | - | - | |
| 0.725741386 | -0.991436958 | -1.366 | - | - | |
| -1.100721598 | -0.751695931 | 0.683 | - | - | |
| 1.704742193 | 0.297694087 | 0.175 | - | - | |

Table 4

Table 4: Numerical value data of methylation patterns of hiPS cells

| P value (set1) | P value (set5) | Ratio r | $S_r$ | Correlation percentage R (%) | Correlation coefficient R' |
|---|---|---|---|---|---|
| -0.09304028 | 0.15528363 | -1.669 | 0 | 0 | 0.303 |
| -0.497550488 | 0.171726614 | -0.345 | 0 | 0 | |
| 0.447785765 | -0.533675909 | -1.192 | 0 | 0 | |
| 0.198727086 | 0.531805515 | 2.676 | 0 | 0 | |
| -0.360826254 | -0.098119058 | 0.272 | 1 | 20 | |
| 0.416845828 | -0.587530494 | -1.409 | 1 | 20 | |
| -0.559108615 | -0.994471312 | 1.779 | 1 | 20 | |
| 0.090189099 | 0.163971722 | 1.818 | - | - | |
| 0.475475103 | 0.395178437 | 0.831 | - | - | |
| 0.578081548 | 0.390057415 | 0.675 | - | - | |
| 0.327318907 | 0.080186345 | 0.245 | - | - | |

With regard to the methylation P values of the line set 1 and the line set 5 shown in Table 1, the ratio r of the P values = set 1/set 5 was obtained.

Subsequently, among the ratio r of the continuous 5 probes (probe group), the ratio whose value is included in 1.3 - 0.7 was selected, the number $S_r$ was then counted, and the correlation percentage R (%) = (Sr/5) x 100 was then obtained.

For example, in Table 1, among the values of the ratio r of the initial probe group (i.e., 0.863, 0.352, 0.444, 0.377 and 0.749), the values of the ratio r that are included in 1.3 - 0.7 (i.e., 0.863 and 0.749) were selected, the number $S_r$ (= 2) was then counted, and the correlation percentage R (%) = (2/5) x 100 = 40% was then calculated. Likewise, with regard to other probe groups, the correlation percentages were calculated.

In Table 1, in individual probe groups enclosed with the frame, the correlation percentages are 80% or more, and thus, it can be said that the probe groups are similar to one another in terms of methylation pattern.

With regard to the patterns of methylation P values in the "set 1" and "set 5" shown in Table 1, the correlation coefficient R' was obtained. As a result, a high value that was 0.920 was obtained.

Likewise, with regard to the methylation P values of the line set 1 and the line set 5 shown in Table 2, the ratio r of the P values = set 1/set 5 was obtained.

Subsequently, among the ratio r of the continuous 5 probes (probe group), the ratio whose value is included in 1.3 - 0.7 was selected, the number $S_r$ was then counted, and the correlation percentage R (%) = ($S_r$/5) x 100 was then obtained.

In Table 2, in individual probe groups enclosed with the frame, the correlation percentages are 80% or more, and thus, the probe groups are similar to one another in terms of methylation pattern.

With regard to the patterns of methylation P values in the "set 1" and "set 5" shown in Table 2, the correlation coefficient R' was obtained. As a result, a high value that was 0.912 was obtained.

With regard to the methylation P values of the line set 1 and the line set 5 shown in Table 3, the ratio r of the P values = set 1/set 5 was obtained.

Subsequently, among the ratio r of the continuous 5 probes (probe group), the ratio whose value is included in 1.3 - 0.7 was selected, the number $S_r$ was then counted, and the correlation percentage R (%) = (Sr/5) x 100 was then obtained.

In Table 3, there were no probe groups in which the correlation percentage was 80% or more.

With regard to the patterns of methylation P values in the "set 1" and "set 5" shown in Table 3, the correlation coefficient R' was obtained. As a result, a value that was 0.701 was obtained.

Moreover, with regard to the methylation P values of the line set 1 and the line set 5 shown in Table 4, the ratio r of the P values = set 1/set 5 was obtained.

Subsequently, among the ratio r of the continuous 5 probes (probe group), the ratio whose value is included in 1.3 - 0.7 was selected, the number $S_r$ was then counted, and the correlation percentage R (%) = (Sr/5) x 100 was then obtained.

In Table 4, there were no probe groups in which the correlation percentage was 80% or more.

With regard to the patterns of methylation P values in the "set 1" and "set 5" shown in Table 4, the correlation coefficient R' was obtained. As a result, a value that was 0.303 was obtained.

[Reference Example 2] Analysis of methylation/hydroxymethylation patterns of ES cells

The above-described Steps 1 to 10 were performed on human ES cells. As a result, the mapping graphs shown in Figures 10 to 13 were obtained.

In each of the mapping graphs shown in Figures 10 to 13, the upper graph shows the pattern of methylation $P_m$ values, and the lower graph shows the pattern of unmethylation $P_{hm}$ values. The graph shown in each of Figures 10 to 13 shows the patterns in the same DNA regions in the human ES cell lines 1 and 2, respectively. In addition, Figures 10 to 13 show the patterns of different DNA regions.

The numerical value data of the methylation P value pattern (5mC) and the hydroxymethylation P value pattern (5hmC) on the mapping graphs of Figures 10 to 13 are shown in Tables 5 to 8, respectively.

Table 5

Table 5: Numerical value data of methylation and hydroxymethylation patterns of human ES cells

| 5mC | | | | | | 5hmC | | | | | |
|---|---|---|---|---|---|---|---|---|---|---|---|
| line A02 | line A03 | Ratio r | $S_r$ | Correlation percentage R (%) | Correlation coefficient R' | line A02 | line A03 | Ratio r | $S_r$ | Correlation percentage R (%) | Correlation coefficient R' |
| 0.01 | 0 | 0.000 | 0 | 0 | 0.953 | 0.19 | 0.03 | 0.16 | 0 | 0 | 0.921 |
| 0.01 | 0 | 0.000 | 0 | 0 | | 0.24 | 0.09 | 0.38 | 0 | 0 | |
| 0.02 | 0 | 0.000 | 0 | 0 | | 0.32 | 0.17 | 0.53 | 0 | 0 | |
| 0.02 | 0 | 0.000 | 1 | 20 | | 0.32 | 0.17 | 0.53 | 0 | 0 | |
| 0.02 | 0 | 0.000 | 2 | 40 | | 0.32 | 0.17 | 0.53 | 1 | 20 | |
| 0.02 | 0.16 | 8.000 | 3 | 60 | | 0.48 | 0.19 | 0.40 | 2 | 40 | |
| 0.04 | 0.16 | 4.000 | 4 | 80 | | 0.48 | 0.17 | 0.35 | 3 | 60 | |
| 0.41 | 0.47 | 1.146 | 5 | 100 | | 1.17 | 0.69 | 0.59 | 4 | 80 | |
| 1.07 | 1.15 | 1.075 | 5 | 100 | | 1.17 | 1.08 | 0.92 | 5 | 100 | |
| 1.07 | 1.15 | 1.075 | 5 | 100 | | 1.17 | 1.08 | 0.92 | 5 | 100 | |
| 1.07 | 1.15 | 1.075 | 5 | 100 | | 1.17 | 1.08 | 0.92 | 5 | 100 | |
| 1.93 | 2.05 | 1.062 | 4 | 80 | | 1.17 | 1.08 | 0.92 | 4 | 80 | |
| 1.93 | 2.05 | 1.062 | 4 | 80 | | 1.17 | 1.08 | 0.92 | 3 | 60 | |
| 1.93 | 2.05 | 1.062 | 3 | 60 | | 1.17 | 1.08 | 0.92 | 2 | 40 | |
| 1.93 | 2.05 | 1.062 | 3 | 60 | | 1.27 | 1.08 | 0.85 | 2 | 40 | |
| 1.87 | 1.2 | 0.642 | 3 | 60 | | 1.12 | 0.51 | 0.46 | 1 | 20 | |
| 2.08 | 1.91 | 0.918 | 4 | 80 | | 1.12 | 0.51 | 0.46 | 1 | 20 | |
| 1.87 | 1.1 | 0.588 | 4 | 80 | | 1.12 | 0.51 | 0.46 | 1 | 20 | |
| 1.02 | 0.76 | 0.745 | 5 | 100 | | 0.52 | 0.42 | 0.81 | 1 | 20 | |
| 1.02 | 0.76 | 0.745 | 4 | 80 | | 0.08 | 0.05 | 0.63 | 0 | 0 | |
| 0.8 | 0.65 | 0.813 | - | - | | 0.01 | 0 | 0.00 | - | - | |
| 0.58 | 0.54 | 0.931 | - | - | | 0 | 0 | #DIV/0! | - | - | |
| 0.37 | 0.43 | 1.162 | - | - | | 0 | 0 | #DIV/0! | - | - | |
| 0.17 | 0.32 | 1.882 | - | - | | 0 | 0 | #DIV/0! | - | - | |

Table 6

Table 6: Numerical value data of methylation and hydroxymethylation patterns of human ES cells

| 5mC | | | | | | 5hmC | | | | | |
|---|---|---|---|---|---|---|---|---|---|---|---|
| line A02 | line A03 | Ratio r | $S_r$ | Correlation percentage R (%) | Correlation coefficient R' | line A02 | line A03 | Ratio r | $S_r$ | Correlation percentage R (%) | Correlation coefficient R' |
| 1.38 | 1.21 | 0.877 | 2 | 40 | 0.973 | 0 | 1.82 | #DIV/0! | 0 | 0 | 0.588 |
| 1.38 | 1.21 | 0.877 | 1 | 20 | | 0 | 1.82 | #DIV/0! | 0 | 0 | |
| 2.14 | 1.21 | 0.565 | 1 | 20 | | 0 | 3.17 | #DIV/0! | 0 | 0 | |
| 2.14 | 1.21 | 0.565 | 2 | 40 | | 0 | 3.17 | #DIV/0! | 0 | 0 | |
| 2.52 | 1.44 | 0.571 | 3 | 60 | | 0.01 | 3.71 | 371.000 | 0 | 0 | |
| 3.23 | 2.2 | 0.681 | 4 | 80 | | 0.92 | 4.24 | 4.609 | 0 | 0 | |
| 3.8 | 3.75 | 0.987 | 5 | 100 | | 2.26 | 4.04 | 1.788 | 1 | 20 | |
| 5.46 | 5.4 | 0.989 | 5 | 100 | | 2.54 | 3.51 | 1.382 | 2 | 40 | |
| 5.88 | 6.15 | 1.046 | 5 | 100 | | 1.82 | 2.87 | 1.577 | 2 | 60 | |
| 4.99 | 5.26 | 1.054 | 4 | 80 | | 1.48 | 2.37 | 1.601 | 2 | 60 | |
| 3.59 | 4.09 | 1.139 | 3 | 60 | | 1.48 | 1.41 | 0.953 | 2 | 40 | |
| 2.41 | 2.83 | 1.174 | 3 | 60 | | 0.77 | 0.66 | 0.857 | 1 | 20 | |
| 1.44 | 1.78 | 1.236 | 2 | 40 | | 0.22 | 0.15 | 0.682 | 0 | 0 | |
| 0.68 | 0.94 | 1.382 | 1 | 20 | | 0.05 | 0 | 0.000 | 0 | 0 | |
| 0.16 | 0.33 | 2.063 | 1 | 20 | | 0 | 0 | #DIV/0! | 0 | 20 | |
| 0.01 | 0.01 | 1.000 | 1 | 20 | | 0 | 0 | #DIV/0! | 0 | 20 | |
| 0.01 | 0 | 0.000 | 0 | 0 | | 0 | 0 | #DIV/0! | 0 | 20 | |
| 0.01 | 0 | 0.000 | 0 | 0 | | 0 | 0 | #DIV/0! | 0 | 0 | |
| 0.02 | 0 | 0.000 | 0 | 0 | | 0.01 | 0 | 0.000 | 0 | 0 | |
| 0.02 | 0 | 0.000 | 0 | 0 | | 0.01 | 0 | 0.000 | 0 | 0 | |
| 0.01 | 0 | 0.000 | - | - | | 0.01 | 0 | 0.000 | - | - | |
| 0.01 | 0 | 0.000 | - | - | | 0 | 0 | #DIV/0! | - | - | |
| 0 | 0 | #DIV/0! | - | - | | 0 | 0 | #DIV/0! | - | - | |
| 0.16 | 0 | 0.000 | - | - | | 0 | 0 | #DIV/0! | - | - | |

Table 7

Table 7: Numerical value data of methylation and hydroxymethylation patterns of human ES cells

| 5mC | | | | | | 5hmC | | | | | |
|---|---|---|---|---|---|---|---|---|---|---|---|
| line A01 | line A03 | Ratio r | $S_r$ | Correlation percentage R (%) | Correlation coefficient R' | line A01 | line A03 | Ratio r | $S_r$ | Correlation percentage R (%) | Correlation coefficient R' |
| 0.01 | 0.76 | 76.000 | 0 | 0 | -0.282 | 0 | 0 | #DIV/0! | 0 | 0 | 0.988 |
| 0 | 0.95 | #DIV/0! | 1 | 20 | | 0 | 0 | #DIV/0! | 1 | 20 | |
| 0.03 | 1.14 | 38.000 | 1 | 20 | | 0 | 0 | #DIV/0! | 2 | 40 | |
| 0.2 | 1.33 | 6.650 | 1 | 20 | | 0 | 0 | #DIV/0! | 3 | 60 | |
| 0.08 | 0.74 | 9.250 | 1 | 20 | | 0 | 0 | #DIV/0! | 4 | 80 | |
| 0.53 | 0.58 | 1.094 | 1 | 20 | | 0.08 | 0.09 | 1.13 | 5 | 100 | |
| 1.23 | 0.23 | 0.187 | 0 | 0 | | 0.13 | 0.13 | 1.00 | 5 | 100 | |
| 0.69 | 0.23 | 0.333 | 0 | 0 | | 0.13 | 0.13 | 1.00 | 4 | 80 | |
| 0.69 | 0.23 | 0.333 | 0 | 0 | | 0.13 | 0.13 | 1.00 | 4 | 80 | |
| 0.17 | 0.23 | 1.353 | 0 | 0 | | 0.13 | 0.13 | 1.00 | 4 | 80 | |
| 0 | 0.23 | #DIV/0! | 0 | 0 | | 0.13 | 0.13 | 1.00 | 3 | 60 | |
| 0 | 0.08 | #DIV/0! | 0 | 0 | | 0.53 | 0.34 | 0.64 | 2 | 40 | |
| 0 | 0 | #DIV/0! | 0 | 0 | | 1.23 | 0.96 | 0.78 | 2 | 40 | |
| 0 | 0 | #DIV/0! | 0 | 0 | | 1.42 | 1.8 | 1.27 | 2 | 40 | |
| 0 | 0 | #DIV/0! | 0 | 0 | | 1.82 | 2.86 | 1.57 | 2 | 40 | |
| 0 | 0 | #DIV/0! | 0 | 0 | | 2.88 | 4.12 | 1.43 | 3 | 60 | |
| 0 | 0 | #DIV/0! | 0 | 0 | | 4.16 | 5.61 | 1.35 | 4 | 80 | |
| 0 | 0 | #DIV/0! | 0 | 0 | | 4.47 | 4.99 | 1.12 | 4 | 80 | |
| 0 | 0 | #DIV/0! | 0 | 0 | | 4.47 | 4.99 | 1.12 | 3 | 60 | |
| 0 | 0 | #DIV/0! | 0 | 0 | | 4.47 | 4.99 | 1.12 | 2 | 40 | |
| 0 | 0 | #DIV/0! | 0 | 0 | | 4.47 | 5.05 | 1.13 | 1 | 20 | |
| 0 | 0 | #DIV/0! | 0 | 0 | | 3.69 | 4.96 | 1.34 | 0 | 0 | |
| 0 | 0 | #DIV/0! | 0 | 0 | | 3.22 | 4.34 | 1.35 | 1 | 20 | |
| 0 | 0 | #DIV/0! | 0 | 0 | | 2.76 | 3.73 | 1.35 | 1 | 20 | |
| 0 | 0 | #DIV/0! | 0 | 0 | | 2.29 | 3.11 | 1.36 | 1 | 20 | |
| 0 | 0 | #DIV/0! | 0 | 0 | | 2.76 | 3.73 | 1.35 | 1 | 20 | |
| 0 | 0.04 | #DIV/0! | 0 | 0 | | 1.42 | 1.31 | 0.92 | 1 | 20 | |
| 0 | 0 | #DIV/0! | 0 | 0 | | 0.54 | 0.03 | 0.06 | 0 | 0 | |
| 0 | 0 | #DIV/0! | 0 | 0 | | 0.1 | 0.01 | 0.10 | 0 | 0 | |

(continued)

| 5mC | | | | | | 5hmC | | | | | |
|---|---|---|---|---|---|---|---|---|---|---|---|
| line A01 | line A03 | Ratio r | $S_r$ | Correlation percentage R (%) | Correlation coefficient R' | line A01 | line A03 | Ratio r | $S_r$ | Correlation percentage R (%) | Correlation coefficient R' |
| 0 | 0.06 | #DIV/0! | 0 | 0 | | 0.03 | 0 | 0.00 | 0 | 0 | |
| 0 | 0 | #DIV/0! | - | - | | 0 | 0 | #DIV/0! | - | - | |
| 0 | 0 | #DIV/0! | - | - | | 0 | 0 | #DIV/0! | - | - | |
| 0 | 0 | #DIV/0! | - | - | | 0.01 | 0 | 0.00 | - | - | |
| 0 | 0 | #DIV/0! | - | - | | 0 | 0 | #DIV/0! | - | - | |

Table 8

Table 8: Numerical value data of methylation and hydroxymethylation patterns of human ES cells

| 5mC | | | | | | 5hmC | | | | | |
|---|---|---|---|---|---|---|---|---|---|---|---|
| line A01 | line A03 | Ratio r | $S_r$ | Correlation percentage R (%) | Correlation coefficient R' | line A01 | line A03 | Ratio r | $S_r$ | Correlation percentage R (%) | Correlation coefficient R' |
| 0.59 | 0.1 | 0.169 | 1 | 20 | 0.796 | 0.03 | 0.14 | 4.67 | 3 | 60 | -0.125 |
| 0.45 | 0.06 | 0.133 | 1 | 20 | | 0.01 | 0.02 | 2.00 | 3 | 60 | |
| 0.01 | 0.01 | 1.000 | 1 | 20 | | 0.29 | 0.28 | 0.97 | 3 | 60 | |
| 0.06 | 0.03 | 0.500 | 0 | 0 | | 0.39 | 0.38 | 0.97 | 2 | 40 | |
| 0.23 | 0.05 | 0.217 | 0 | 0 | | 0.49 | 0.48 | 0.98 | 1 | 20 | |
| 0.44 | 0.07 | 0.159 | 0 | 0 | | 0.59 | 0.09 | 0.15 | 0 | 0 | |
| 0.69 | 0.1 | 0.145 | 0 | 0 | | 0.7 | 0.03 | 0.04 | 0 | 0 | |
| 1.16 | 0.07 | 0.060 | 1 | 20 | | 0.59 | 0.02 | 0.03 | 0 | 0 | |
| 1.16 | 0.46 | 0.397 | 1 | 20 | | 0.59 | 0.02 | 0.03 | 0 | 0 | |
| 1.84 | 0.61 | 0.332 | 1 | 20 | | 0.63 | 0.01 | 0.02 | 0 | 0 | |
| 1.84 | 0.92 | 0.500 | - | - | | 1.26 | 0 | 0.00 | - | - | |
| 2.41 | 2.25 | 0.934 | - | - | | 0.78 | 0 | 0.00 | - | - | |
| 0.09 | 0.23 | 2.556 | - | - | | 0 | 0 | #DIV/0! | - | - | |
| 0.35 | 0.65 | 1.857 | - | - | | 0 | 0 | #DIV/0! | - | - | |

The numerical values shown in the "line A02" and "line A03," in the columns "5mC" and "5hmC" in Table 5, indicate the methylation P value and the hydroxymethylation P value, respectively.

Regarding the methylation P values of individual probes, the ratio r of the P values = line A02/line A03 was obtained. Likewise, regarding the hydroxymethylation P values of individual probes, the ratio r of the P values = line A02/line A03 was obtained.

Subsequently, from the methylation r values and the hydroxymethylation r values, those included in the range of 1.3 - 0.7 were selected, the number Sr was then counted, and the correlation percentage R (%) = ($S_r$/5) x 100 was then obtained. The correlation percentages R of both methylation and hydroxymethylation are shown.

In the methylation pattern (5mC) shown in Table 5, in individual probe groups enclosed with the frame, the correlation percentages are 80% or more, and thus, it can be said that the probe groups are similar to one another in terms of methylation pattern.

Moreover, in the hydroxymethylation pattern (5hmC) shown in Table 5, in individual probe groups enclosed with the frame, the correlation percentages are 80% or more, and thus, it can be said that the probe groups are similar to one another in terms of hydroxymethylation pattern.

With regard to the patterns of methylation P values shown in Table 5, the correlation coefficient R' was obtained between the line A02 and the line A03. As a result, a high value that was 0.953 was obtained. With regard to the patterns of hydroxymethylation P values, the value that was the correlation coefficient R' = 0.921 was obtained.

The columns "5mC" and "5hmC" in Table 6 indicate methylation and hydroxymethylation, respectively. In addition, the numerical values shown in the "line A02" and "line A03," in the columns "5mC" and "5hmC" in Table 6, indicate the methylation P value and the hydroxymethylation P value, respectively.

Regarding the methylation P values of individual probes, the ratio r of the P values = line A02/line A03 was obtained. Likewise, regarding the hydroxymethylation P values of individual probes, the ratio r of the P values = line A02/line A03 was obtained.

Subsequently, from the methylation r values and the hydroxymethylation r values, those included in the range of 1.3 - 0.7 were selected, the number Sr was then counted, and the correlation percentage R (%) = ($S_r$/5) x 100 was then obtained.

In the methylation pattern shown in Table 6, in individual probe groups enclosed with the frame, the correlation percentages are 80% or more, and thus, it can be said that the probe groups are similar to one another in terms of methylation pattern.

On the other hand, in the hydroxymethylation pattern shown in Table 6, there were found no probe groups in which the correlation percentages were 80% or more.

With regard to the patterns of methylation P values shown in Table 6, the correlation coefficient R' was obtained between the line A02 and the line A03. As a result, a high value that was 0.973 was obtained. With regard to the patterns of hydroxymethylation P values, the value that was the correlation coefficient R' = 0.588 was obtained.

The columns "5mC" and "5hmC" in Table 7 indicate methylation and hydroxymethylation, respectively. In addition, the numerical values shown in the "line A01" and "line A03," in the columns "5mC" and "5hmC" in Table 7, indicate the methylation P value and the hydroxymethylation P value, respectively.

Regarding the methylation P values of individual probes, the ratio r of the P values = line A01/line A03 was obtained. Likewise, regarding the hydroxymethylation P values of individual probes, the ratio r of the P values = line A01/line A03 was obtained.

Subsequently, from the methylation r values and the hydroxymethylation r values, those included in the range of 1.3 - 0.7 were selected, the number Sr was then counted, and the correlation percentage R (%) = ($S_r$/5) x 100 was then obtained.

In the methylation pattern shown in Table 7, there were found no probe groups in which the correlation percentages were 80% or more.

On the other hand, in the hydroxymethylation pattern shown in Table 7, in individual probe groups enclosed with the frame, the correlation percentages are 80% or more, and thus, it can be said that the probe groups are similar to one another in terms of hydroxymethylation pattern.

With regard to the patterns of methylation P values shown in Table 7, the correlation coefficient R' was obtained between the line A01 and the line A03. As a result, a value that was -0.282 was obtained. With regard to the patterns of hydroxymethylation P values, the value that was the correlation coefficient R' = 0.988 was obtained.

The columns "5mC" and "5hmC" in Table 8 indicate methylation and hydroxymethylation, respectively. In addition, the numerical values shown in the "line A01" and "line A03," in the columns "5mC" and "5hmC" in Table 8, indicate the methylation P value and the hydroxymethylation P value, respectively.

Regarding the methylation P values of individual probes, the ratio r of the P values = line A01/line A03 was obtained. Likewise, regarding the hydroxymethylation P values of individual probes, the ratio r of the P values = line A01/line A03 was obtained.

Subsequently, from the methylation r values and the hydroxymethylation r values, those included in the range

of 1.3 - 0.7 were selected, the number Sr was then counted, and the correlation percentage R (%) = ($S_r$/5) x 100 was then obtained.

In the methylation pattern shown in Table 8, there were found no probe groups in which the correlation percentages were 80% or more. Likewise, in the hydroxymethylation pattern shown in Table 8 as well, there were found no probe groups in which the correlation percentages were 80% or more.

With regard to the patterns of methylation P values shown in Table 8, the correlation coefficient R' was obtained between the line A01 and the line A03. As a result, a value that was 0.796 was obtained. With regard to the patterns of hydroxymethylation P values, the value that was the correlation coefficient R' = -0.125 was obtained.

[Reference Example 3] Identification method using correlation coefficient R'

Steps 1 to 9 as described in the above Experimental Procedures were performed on three ES cell lines (A01 to A03), so as to obtain the mapping graphs of methylation $P_m$ values (Figure 14). Cases 1 to 3 of Figure 14 show mapping graphs regarding different DNA regions. The correlation coefficient R' between cell groups was obtained from the P values of methylcytosine modification, using a probe set consisting of 10 to 20 probes as a unit. It was determined that cell groups having a correlation coefficient R' = 0.8 to 1.0 were similar to each other.

Using Case 1 of Figure 14, a determination method will be explained. For each set of 10 to 20 continuous probes, the correlation coefficients between the P values of, the line 1 and the line 2, the line 2 and the line 3, and the line 3 and the line 1, were obtained. Three framed portions shown in Figure 15 are the numerical data of the P values in each of Cases 1, 2 and 3.

As shown in the following Table 9, the correlation coefficients were calculated to be 0.29, 0.94 and 0.17, respectively. There was observed similarity between the line 2 and the line 3 in terms of an increase tendency of the histogram of P values. It was confirmed based on the correlation coefficients that the line 2 and the line 3 were similar to each other in terms of an increase pattern of the P values. Specific numerical value data are shown in Figure 15.

In Case 2, the correlation coefficients were calculated to be 0.24, 0.11 and -0.34, and there was found no similarity in the histogram of P values.

Likewise, in Case 3, the correlation coefficient was calculated to be 0.94 between the line 3 and the line 1. Moreover, it could be confirmed that the patterns were also similar to each other. On the other hand, between the line 1 and the line 2, and between the line 2 and the line 3, the correlation coefficients were -0.12 and -0.14, respectively, and there was not found any similarity in an increase pattern of the P values.

Table 9

Table 9

| | r (A01 vs A02) | r (A02vs A03) | r (A03 vs A01) |
|---|---|---|---|
| Case 1 | 0.29 | 0.94 | 0.17 |
| Case 2 | 0.24 | 0.11 | -0.34 |
| Case 3 | -0.12 | -0.14 | 0.94 |

Industrial Applicability

According to the method of the present invention, it has become possible to more simply and clearly identifying the properties of stem cells by comparing the P value patterns of methylation with those of demethylation. In addition, it has been demonstrated that, upon application of the method of the present invention, operations required for the experiment can be simplified and data with high reproducibility and reliability can be obtained by using the automatic epigenetics system that has been developed by the present inventors.

## Claims

**1.** A method for identifying stem cells, which comprises analyzing the patterns of methylation and demethylation of chromosomal DNA extracted from test stem cells, comparing the analytical results with the methylation and demethylation states with reference stem cells, and correlating the analyzed patterns with the properties of the test stem cells; wherein the properties of stem cells are used to characterize the stage of passage or differentiation;
wherein the analysis of the patterns of methylation and demethylation is carried out by methylated DNA immunoprecipitation (MeDIP) and hydroxymethylated DNA immunoprecipitation (hMeDIP), a hybridization treatment using

a microarray, a treatment of probability values of signal data obtained by the hybridization treatment, and the mapping of the probability values; and
wherein the mapping of the probability values comprises:

(1) a step of assigning the methylation probability values $P_m$ and demethylation probability values $P_{hm}$ of individual probes on a microarray to probe numbers which correspond to the positions on the chromosome,
(2) a step of selecting the probe number n (wherein n is an integer) of a test stem cell and the microarray probe number n' (wherein n' is an integer) of a reference stem cell corresponding to the probe number n,
(3) a step of obtaining the ratios ($r_m = P_{m(n)} / P_{m(n')}$ and $r_{hm} = P_{hm(n)} / P_{hm(n')}$) of the probability values $P_{m(n)}$ and $P_{hm(n)}$ assigned to the probe number n to the probability values $P_{m(n')}$ and $P_{hm(n')}$ assigned to the probe number n',
(4) a step of repeating the step (1) from the continuous probe numbers n to n + i (wherein i is an integer) and then counting the number $S_r$ of the ratio $r_m$ that is in the range of 0.5 to 1.5 and the number $S_r$ of $r_{hm}$ that is in the range of 0.5 to 1.5, and
(5) regarding each of the methylation probability values $R_m$ and the demethylation probability values $R_{hm}$, a step of obtaining the following correlation percentage:

$$R (\%) = \{S_r / (i + 1)\} \text{ x } 100.$$

**2.** The method according to claim 1, wherein the analysis of the patterns of methylation and demethylation is carried out using automated equipment.

**3.** The method according to claim 1, wherein the stem cells are ES cells or iPS cells.

**4.** The method according to claim 1, wherein the stem cells are ectodermal, endodermal or mesodermal stem cells.

**5.** The method according to claim 1, wherein the correlation of the analyzed patterns with the properties of the test stem cells is carried out based on the following criteria (a) to (d):

(a) when the methylation correlation percentage $R_m$ is 70% or more and the demethylation correlation percentage $R_{hm}$ is 70% or more, the test stem cells and the reference stem cells are in a similar state in terms of both methylation and demethylation,
(b) when the methylation correlation percentage $R_m$ is less than 70% and the demethylation correlation percentage $R_{hm}$ is 70% or more, the test stem cells and the reference stem cells are in a similar state in terms of demethylation, but are in a dissimilar state in terms of methylation,
(c) when the methylation correlation percentage $R_m$ is 70% or more and the demethylation correlation percentage $R_{hm}$ is less than 70%, the test stem cells and the reference stem cells are in a similar state in terms of methylation, but are in a dissimilar state in terms of demethylation, and
(d) when the methylation correlation percentage $R_m$ is less than 70% and the demethylation correlation percentage $R_{hm}$ is less than 70%, the test stem cells and the reference stem cells are in a dissimilar state in terms of both methylation and demethylation.

**6.** The method according to claim 1, wherein the correlation of the analyzed patterns with the properties of the test stem cells is carried out based on the following criteria (a') to (d'):

(a') when the methylation correlation coefficient $R'_m$ is 0.7 or more and the demethylation correlation coefficient $R'_{hm}$ is 0.7 or more, the test stem cells and the reference stem cells are in a similar state in terms of both methylation and demethylation,
(b') when the methylation correlation coefficient $R'_m$ is less than 0.7 and the demethylation correlation coefficient $R'_{hm}$ is 0.7 or more, the test stem cells and the reference stem cells are in a similar state in terms of demethylation, but are in a dissimilar state in terms of methylation,
(c') when the methylation correlation coefficient $R'_m$ is 0.7 or more and the demethylation correlation coefficient $R'_{hm}$ is less than 0.7, the test stem cells and the reference stem cells are in a similar state in terms of methylation, but are in a dissimilar state in terms of demethylation, and
(d') when the methylation correlation coefficient $R'_m$ is less than 0.7 and the demethylation correlation coefficient $R'_{hm}$ is less than 0.7, the test stem cells and the reference stem cells are in a dissimilar state in terms of both methylation and demethylation.

**Patentansprüche**

1. Verfahren zum Identifizieren von Stammzellen, das das Analysieren der Methylierungs- und Demethylierungsmuster von chromosomaler DNA, die von Teststammzellen extrahiert wird, das Vergleichen der Analyseergebnisse mit den Methylierungs- und Demethylierungszuständen mit Referenzstammzellen, und das Zuordnen der analysierten Muster zu den Eigenschaften der Teststammzellen umfasst;
wobei die Eigenschaften von Stammzellen verwendet werden, um die Durchgangs- oder Differenzierungsphase zu charakterisieren;
wobei die Analyse der Methylierungs- und Demethylierungsmuster durch methylierte DNA-Immunpräzipitation (MeDIP) und hydromethylierte DNA-Immunpräzipitation (hMeDIP), eine Hybridisierungsbehandlung unter Verwendung eines Mikroarrays, eine Behandlung von Wahrscheinlichkeitswerten von Signaldaten, die durch die Hybridisierungsbehandlung erhalten werden, und dem Kartieren der Wahrscheinlichkeitswerte durchgeführt wird; und
wobei das Kartieren der Wahrscheinlichkeitsdaten umfasst:

(1) einen Schritt des Zuordnens der Methylierungs-Wahrscheinlichkeitswerte $P_m$ und Demethylierungs-Wahrscheinlichkeitswerte $P_{hm}$ von Einzelsonden auf einem Mikroarray zum Sondieren von Zahlen, die den Positionen auf dem Chromosom entsprechen,
(2) einen Schritt des Auswählens der Sondenzahl n (wobei n eine ganze Zahl ist) einer Teststammzelle und der Mikroarray-Sondenzahl n' (wobei n' eine ganze Zahl ist) einer Referenzstammzelle entsprechend der Sondenzahl n,
(3) einen Schritt des Erhaltens der Verhältnisse ($r_m = P_{m(n)}/ P_{m(n')}$ und $r_{hm} = P_{hm(n)}/ P_{hm(n')}$) der Wahrscheinlichkeitswerte $P_{m(n)}$ und $P_{hm(n)}$, die der Sondenzahl n zugeordnet sind, zu den Wahrscheinlichkeitswerten $P_{m(n')}$ und $P_{hm(n')}$, die der Sondenzahl n' zugeordnet sind,
(4) einen Schritt des Wiederholens von Schritt (1) von den fortlaufenden Zahlen n bis n + i (wobei i eine ganze Zahl ist) und dann Zählen der Zahl $S_r$ des Verhältnisses $r_m$, das im Bereich von 0,5 bis 1,5 liegt, und der Zahl $S_r$ von $r_{hm}$, die im Bereich von 0,5 bis 1,5 liegt, und
(5) bezüglich jedes der Methylierungs-Wahrscheinlichkeitswerte $R_m$ und der Demethylierungs-Wahrscheinlichkeitswerte $R_{hm}$ einen Schritt des Erhaltens des folgenden Korrelationsprozentsatzes:

$$R\,(\%) = \{S_r / (i + 1)\} \times 100.$$

2. Verfahren nach Anspruch 1, wobei die Analyse der Methylierungs- und Demethylierungsmuster unter Verwendung einer automatisierten Anlage durchgeführt wird.

3. Verfahren nach Anspruch 1, wobei die Stammzellen ES-Zellen oder iPS-Zellen sind.

4. Verfahren nach Anspruch 1, wobei die Stammzellen ektodermale, endodermale oder mesodermale Stammzellen sind.

5. Verfahren nach Anspruch 1, wobei die Korrelation der analysierten Muster mit den Eigenschaften der Teststammzellen basierend auf folgenden Kriterien (a) bis (d) durchgeführt wird:

(a) wenn der Methylierungs-Korrelations-Prozentsatz $R_m$ 70 % oder mehr ist und der Demethylierungs-Korrelations-Prozentsatz $R_{hm}$ 70 % oder mehr ist, sind die Teststammzellen und die Referenzstammzellen in einem ähnlichen Zustand bezüglich der Methylierung und Demethylierung,
(b) wenn der Methylierungs-Korrelations-Prozentsatz $R_m$ weniger als 70 % ist und der Demethylierungs-Korrelations-Prozentsatz $R_{hm}$ 70 % oder mehr ist, sind die Teststammzellen und die Referenzstammzellen in einem ähnlichen Zustand bezüglich der Demethylierung, sind jedoch in einem unterschiedlichen Zustand bezüglich der Methylierung,
(c) wenn der Methylierungs-Korrelations-Prozentsatz $R_m$ 70 % oder mehr ist und der Demethylierungs-Korrelations-Prozentsatz $R_{hm}$ weniger als 70 % ist, sind die Teststammzellen und die Referenzstammzellen in einem ähnlichen Zustand bezüglich der Methylierung, sind jedoch in einem unterschiedlichen Zustand bezüglich der Demethylierung, und
(d) wenn der Methylierungs-Korrelations-Prozentsatz $R_m$ weniger als 70 % ist und der Demethylierungs-Korrelations-Prozentsatz $R_{hm}$ weniger als 70 % ist, sind die Teststammzellen und die Referenzstammzellen in einem ähnlichen Zustand bezüglich der Methylierung und Demethylierung.

6. Verfahren nach Anspruch 1, wobei die Korrelation der analysierten Muster mit den Eigenschaften der Teststammzellen basierend auf den folgenden Kriterien (a') bis (d') durchgeführt wird:

(a') wenn der Methylierungs-Korrelations-Koeffizient $R'_m$ 0,7 oder mehr ist und der Demethylierungs-Korrelations-Koeffizient $R'_{hm}$ 0,7 oder mehr ist, sind die Teststammzellen und die Referenzstammzellen in einem ähnlichen Zustand bezüglich der Methylierung und Demethylierung,
(b') wenn der Methylierungs-Korrelations-Koeffizient $R'_m$ weniger als 0,7 ist und der Demethylierungs-Korrelations-Koeffizient $R'_{hm}$ 0,7 oder mehr ist, sind die Teststammzellen und die Referenzstammzellen in einem ähnlichen Zustand bezüglich der Demethylierung, sind jedoch in einem unterschiedlichen Zustand bezüglich der Methylierung,
(c') wenn der Methylierungs-Korrelations-Koeffizient $R'_m$ 0,7 oder mehr ist und der Demethylierungs-Korrelations-Koeffizient $R'_{hm}$ weniger als 0,7 ist, sind die Teststammzellen und die Referenzstammzellen in einem ähnlichen Zustand bezüglich der Methylierung, sind jedoch in einem unterschiedlichen Zustand bezüglich der Demethylierung. und
(d') wenn der Methylierungs-Korrelations-Koeffizient $R'_m$ weniger als 0,7 ist und der Demethylierungs-Korrelations-Koeffizient $R'_{hm}$ weniger als 0,7 ist, sind die Teststammzellen und die Referenzstammzellen in einem unterschiedlichen Zustand bezüglich der Methylierung und Demethylierung.

## Revendications

1. Procédé pour identifier des cellules souches, qui comprend l'analyse des schémas de méthylation et de déméthylation d'ADN chromosomique extrait de cellules souches d'essai, la comparaison des résultats analytiques aux états de méthylation et de déméthylation avec des cellules souches de référence, et la corrélation des motifs analysés avec les propriétés des cellules souches d'essai ;
dans lequel les propriétés de cellules souches sont utilisées pour caractériser le stade de passage ou de différentiation ;
dans lequel l'analyse des schémas de méthylation et de déméthylation est réalisée par immunoprécipitation d'ADN méthylé (MeDIP) et immunoprécipitation d'ADN hydroxyméthylé (hMeDIP), un traitement d'hybridation en utilisant un microréseau, un traitement de valeurs de probabilité de données de signal obtenues par le traitement d'hybridation, et le mappage des valeurs de probabilité ; et
dans lequel le mappage des valeurs de probabilité comprend :

(1) une étape de l'attribution des valeurs de probabilité de méthylation $P_m$ et des valeurs de probabilité de déméthylation $P_{hm}$ de sondes individuelles sur un microréseau à des nombres de sondes qui correspondent aux positions sur le chromosome,
(2) une étape de la sélection du nombre de sondes n (n étant un nombre entier relatif) d'une cellule souche d'essai et du nombre de sondes de microréseau n' (n' étant un nombre entier relatif) d'une cellule souche de référence correspondant au nombre de sondes n,
(3) une étape de l'obtention des rapports ($r_m$ = Pm(n) / Pm(n') et $r_{hm} = P_{hm(n)} / P_{hm(n')}$) des valeurs de probabilité $P_{m(n)}$ et $P_{hm(n)}$ attribuées au nombre de sondes n par rapport aux valeurs de probabilité $P_{m(n')}$ et $P_{hm(n')}$ attribuées au nombre de sondes n',
(4) une étape de la répétition de l'étape (1) des nombres de sondes continus n à n + i (i étant un nombre entier relatif) et puis du comptage du nombre $S_r$ du rapport $r_m$ qui est dans la plage de 0,5 à 1,5 et du nombre $S_r$ de $r_{hm}$ qui est dans la plage de 0,5 à 1,5, et
(5) concernant chacune des valeurs de probabilité de méthylation $R_m$ et des valeurs de probabilité de déméthylation $R_{hm}$, une étape de l'obtention du pourcentage de corrélation suivant :

$$R\ (\%) = \{Sr/(i+1)\} \times 100.$$

2. Procédé selon la revendication 1, dans lequel l'analyse des schémas de méthylation et de déméthylation est réalisée en utilisant un équipement automatisé.

3. Procédé selon la revendication 1, dans lequel les cellules souches sont des cellules ES ou des cellules iPS.

4. Procédé selon la revendication 1, dans lequel les cellules souches sont des cellules souches ectodermiques, endodermiques ou mésodermiques.

**5.** Procédé selon la revendication 1, dans lequel la corrélation des motifs analysés avec les propriétés des cellules souches d'essai est réalisée en fonction des critères suivants (a) à (d) :

(a) lorsque le pourcentage de corrélation de méthylation $R_m$ est 70 % ou plus et le pourcentage de corrélation de déméthylation $R_{hm}$ est 70 % ou plus, les cellules souches d'essai et les cellules souches de référence sont dans un état similaire en termes de méthylation et de déméthylation,
(b) lorsque le pourcentage de corrélation de méthylation $R_m$ est inférieur à 70 % et le pourcentage de corrélation de déméthylation $R_{hm}$ est 70 % ou plus, les cellules souches d'essai et les cellules souches de référence sont dans un état similaire en termes de déméthylation, mais sont dans un état dissimilaire en termes de méthylation,
(c) lorsque le pourcentage de corrélation de méthylation $R_m$ est 70 % ou plus et le pourcentage de corrélation de déméthylation $R_{hm}$ est inférieur à 70 %, les cellules souches d'essai et les cellules souches de référence sont dans un état similaire en termes de méthylation, mais sont dans un état dissimilaire en termes de déméthylation, et
(d) lorsque le pourcentage de corrélation de méthylation $R_m$ est inférieur à 70 % et le pourcentage de corrélation de déméthylation $R_{hm}$ est inférieur à 70 %, les cellules souches d'essai et les cellules souches de référence sont dans un état dissimilaire en termes de méthylation et de déméthylation.

**6.** Procédé selon la revendication 1, dans lequel la corrélation des schémas analysés avec les propriétés des cellules souches d'essai est réalisée en fonction des critères suivants (a') à (d') :

(a') lorsque le coefficient de corrélation de méthylation $R'_m$ est 0,7 ou plus et le coefficient de corrélation de déméthylation $R'_{hm}$ est 0,7 ou plus, les cellules souches d'essai et les cellules souches de référence sont dans un état similaire en termes de méthylation et de déméthylation,
(b') lorsque le coefficient de corrélation de méthylation $R'_m$ est inférieur à 0,7 et le coefficient de corrélation de déméthylation $R'_{hm}$ est 0,7 ou plus, les cellules souches d'essai et les cellules souches de référence sont dans un état similaire en termes de déméthylation, mais sont dans un état dissimilaire en termes de méthylation,
(c') lorsque le coefficient de corrélation de méthylation $R'_m$ est 0,7 ou plus et le coefficient de corrélation de déméthylation $R'_{hm}$ est inférieur à 0,7, les cellules souches d'essai et les cellules souches de référence sont dans un état similaire en termes de méthylation, mais sont dans un état dissimilaire en termes de déméthylation, et
(d') lorsque le coefficient de corrélation de méthylation $R'_m$ est inférieur à 0,7 et le coefficient de corrélation de déméthylation $R'_{hm}$ est inférieur à 0,7, les cellules souches d'essai et les cellules souches de référence sont dans un état dissimilaire en termes de méthylation et de déméthylation.

# Figure 1

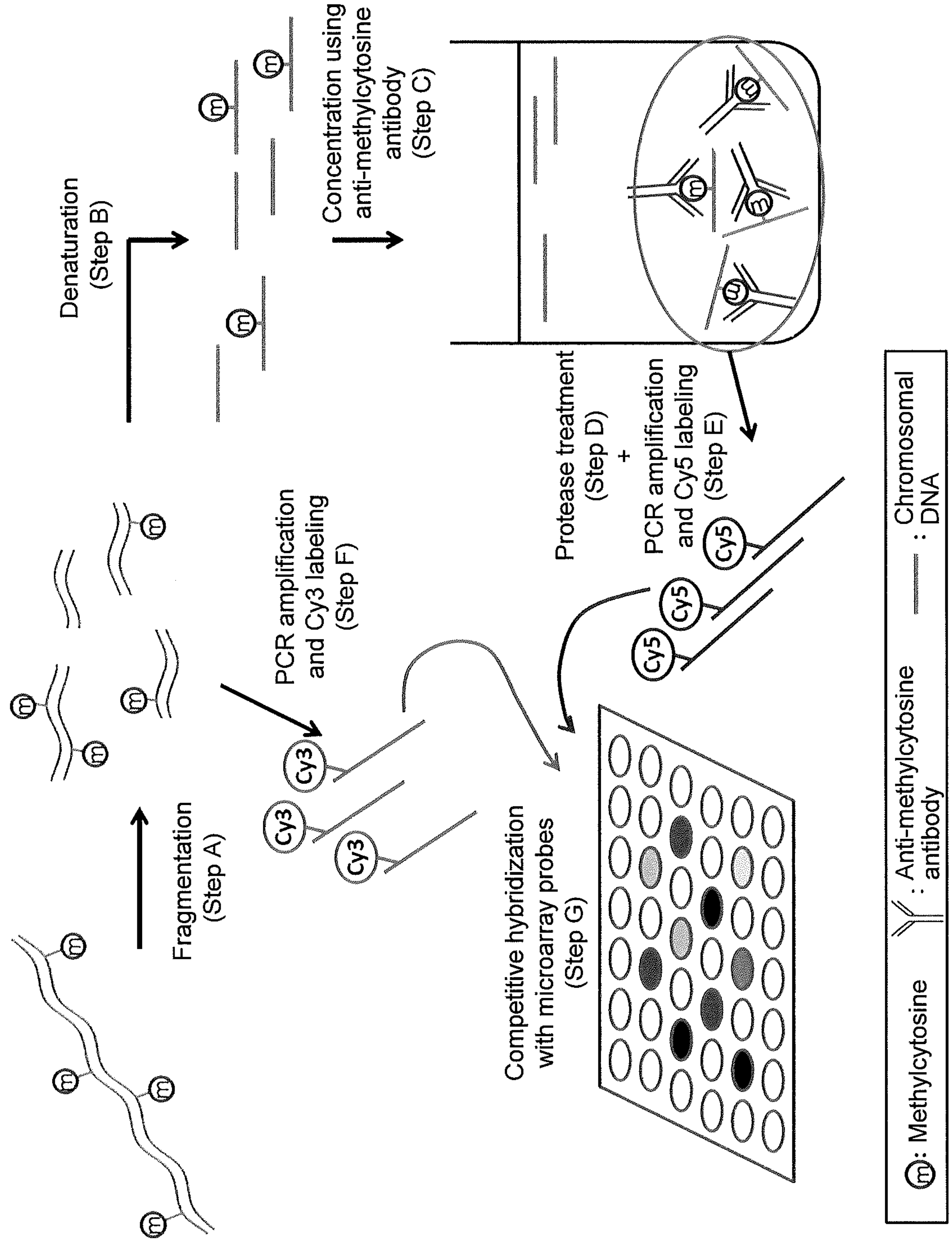
Fragmentation
(Step A)
Denaturation
(Step B)
Concentration using
anti-methylcytosine
antibody
(Step C)
Protease treatment
(Step D)
+
PCR amplification
and Cy5 labeling
(Step E)
PCR amplification
and Cy3 labeling
(Step F)
Competitive hybridization
with microarray probes
(Step G)
Cy3
Cy5
m: Methylcytosine
: Anti-methylcytosine antibody
: Chromosomal DNA

Figure 2

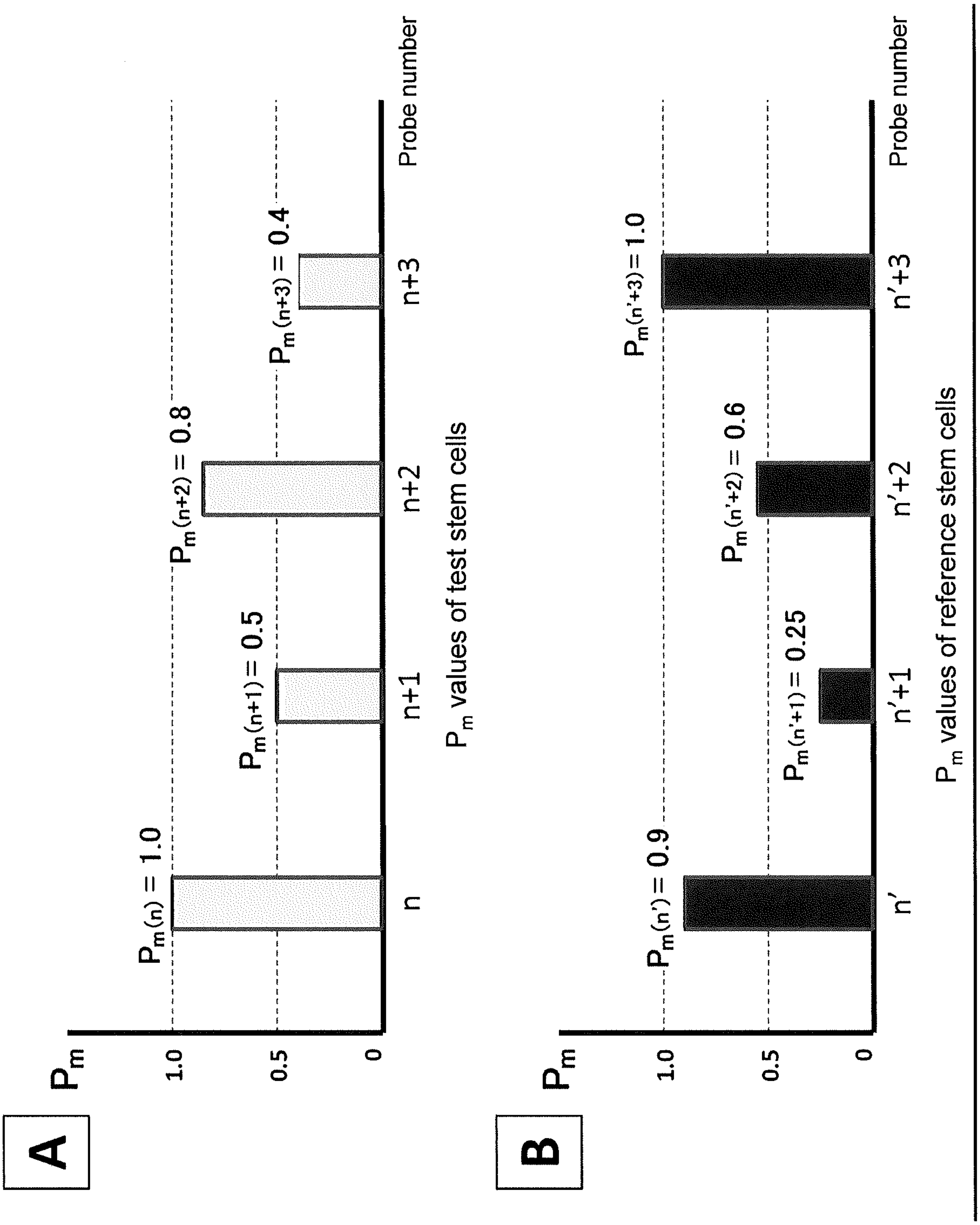
A
$P_m$
1.0
0.5
0
$P_m(n) = 1.0$
$P_m(n+1) = 0.5$
$P_m(n+2) = 0.8$
$P_m(n+3) = 0.4$
n
n+1
n+2
n+3
Probe number
$P_m$ values of test stem cells
B
$P_m$
1.0
0.5
0
$P_m(n') = 0.9$
$P_m(n'+1) = 0.25$
$P_m(n'+2) = 0.6$
$P_m(n'+3) = 1.0$
n'
n'+1
n'+2
n'+3
Probe number
$P_m$ values of reference stem cells

Figure 3

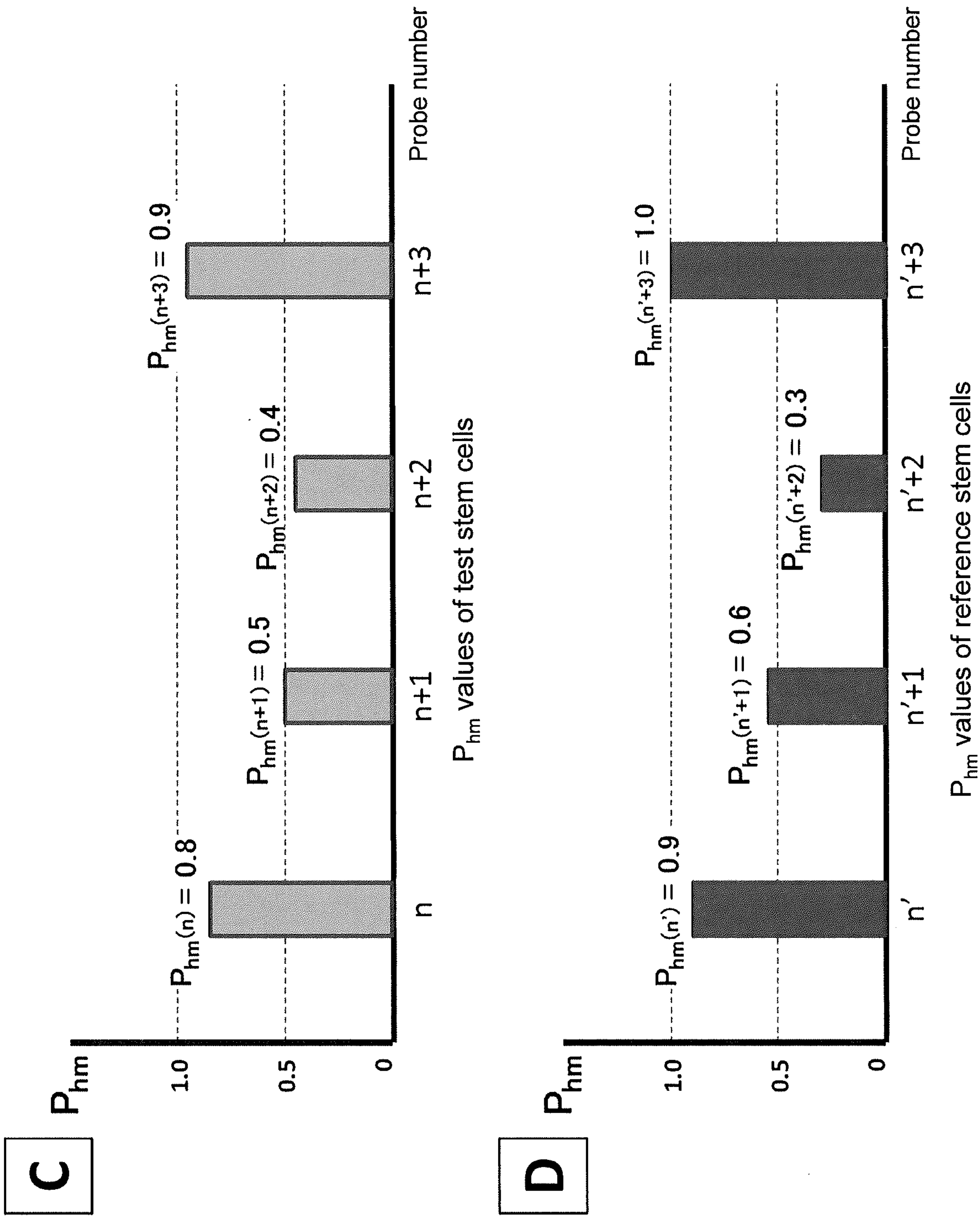
C
$P_{hm}$
1.0
0.5
0
$P_{hm}(n) = 0.8$
$P_{hm}(n+1) = 0.5$
$P_{hm}(n+2) = 0.4$
$P_{hm}(n+3) = 0.9$
n
n+1
n+2
n+3
Probe number
$P_{hm}$ values of test stem cells
D
$P_{hm}$
1.0
0.5
0
$P_{hm}(n') = 0.9$
$P_{hm}(n'+1) = 0.6$
$P_{hm}(n'+2) = 0.3$
$P_{hm}(n'+3) = 1.0$
n'
n'+1
n'+2
n'+3
Probe number
$P_{hm}$ values of reference stem cells

# Figure 4

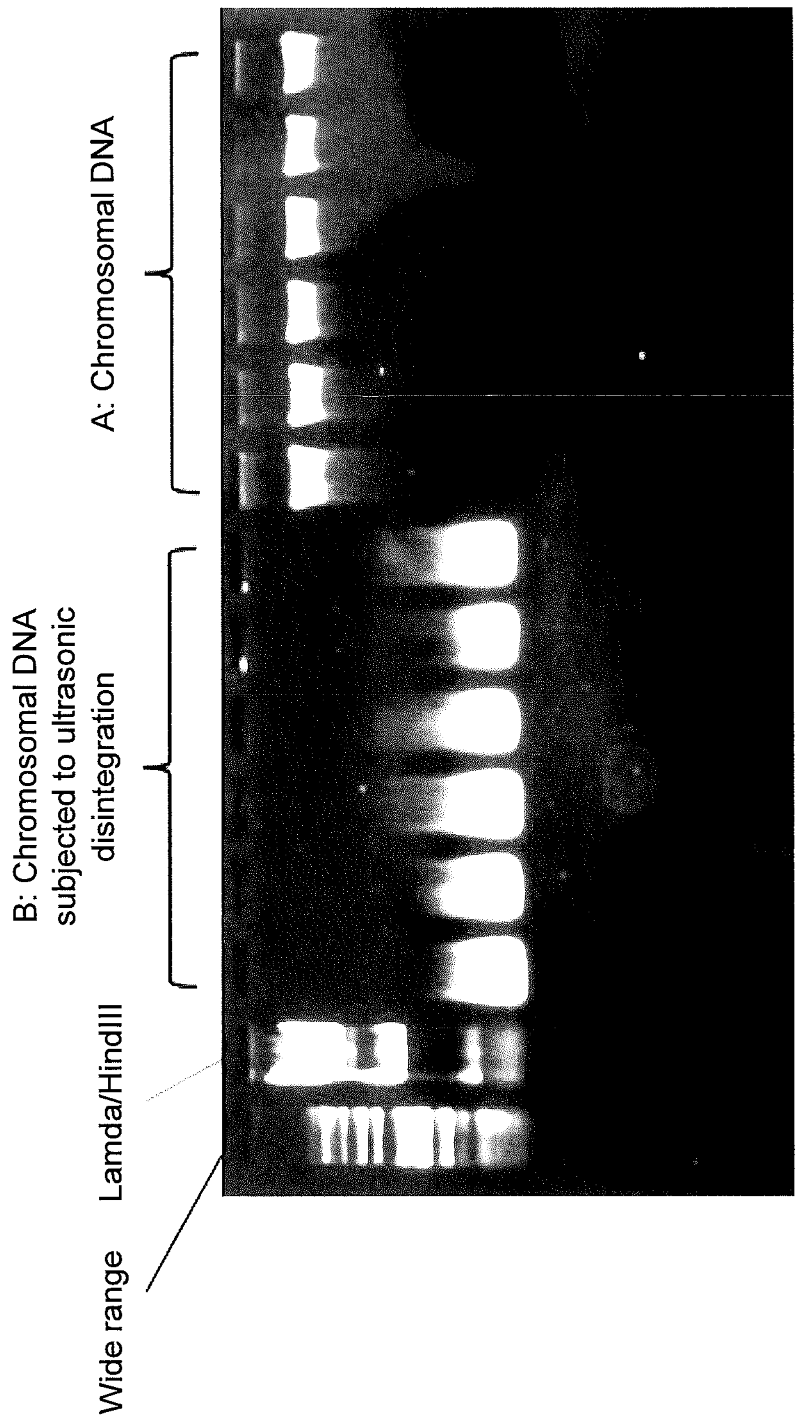
A: Chromosomal DNA
B: Chromosomal DNA subjected to ultrasonic disintegration
Lamda/HindIII
Wide range

Figure 5

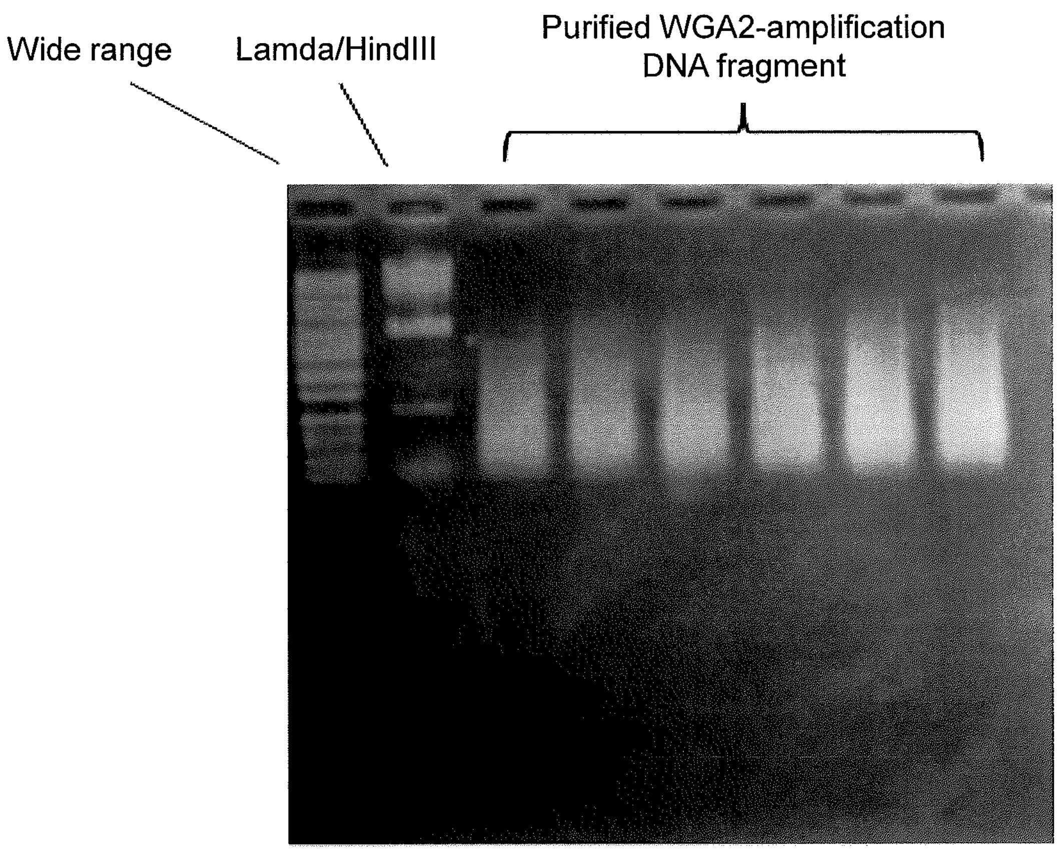
Wide range
Lamda/HindIII
Purified WGA2-amplification
DNA fragment

# Figure 6

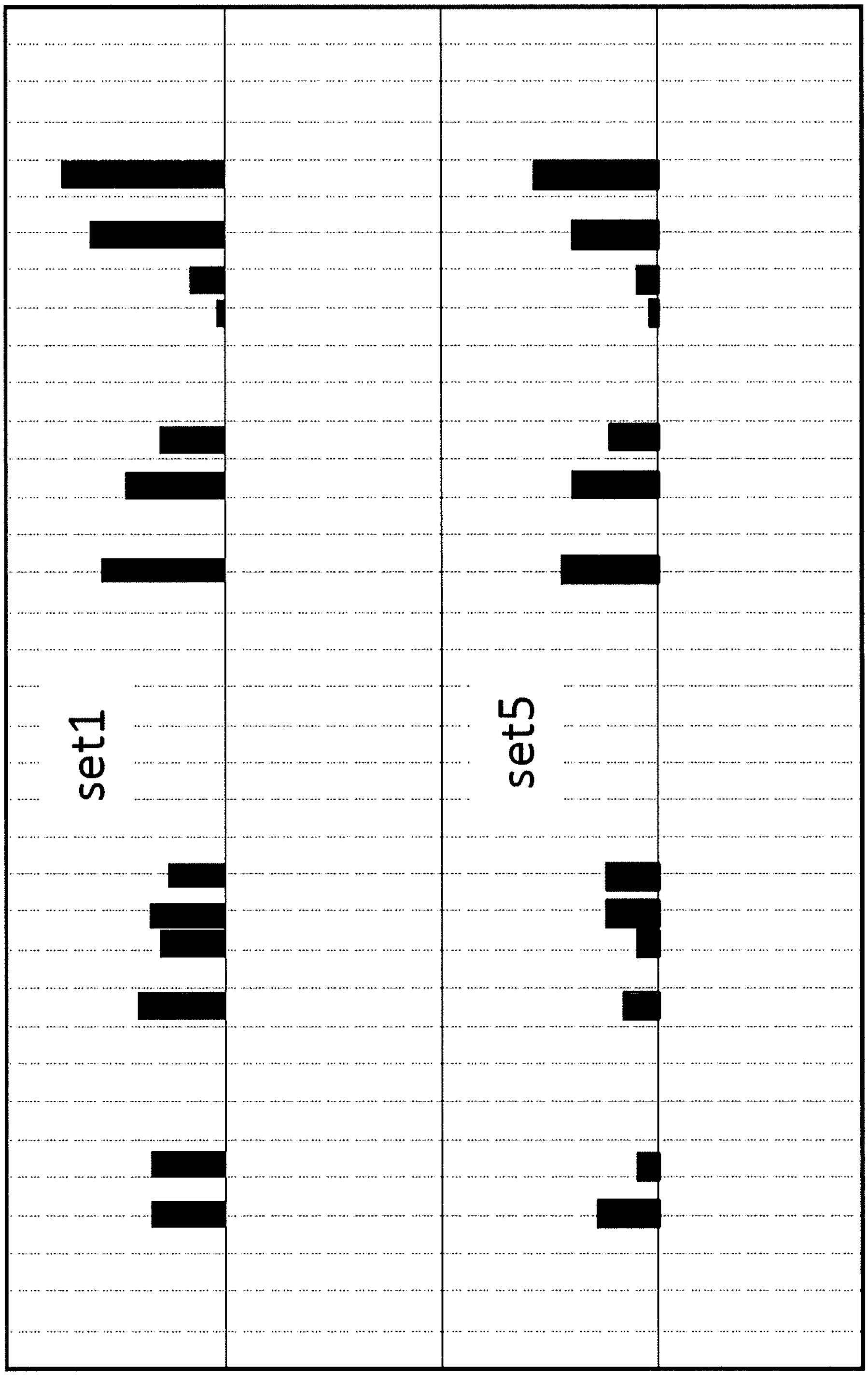

# Figure 7

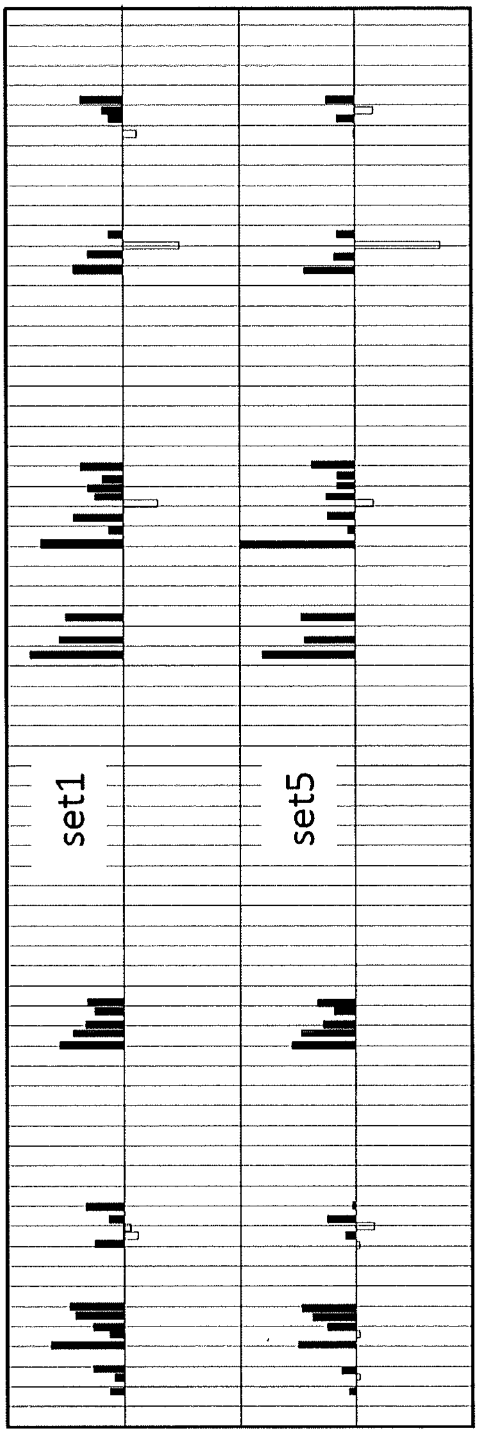

# Figure 8

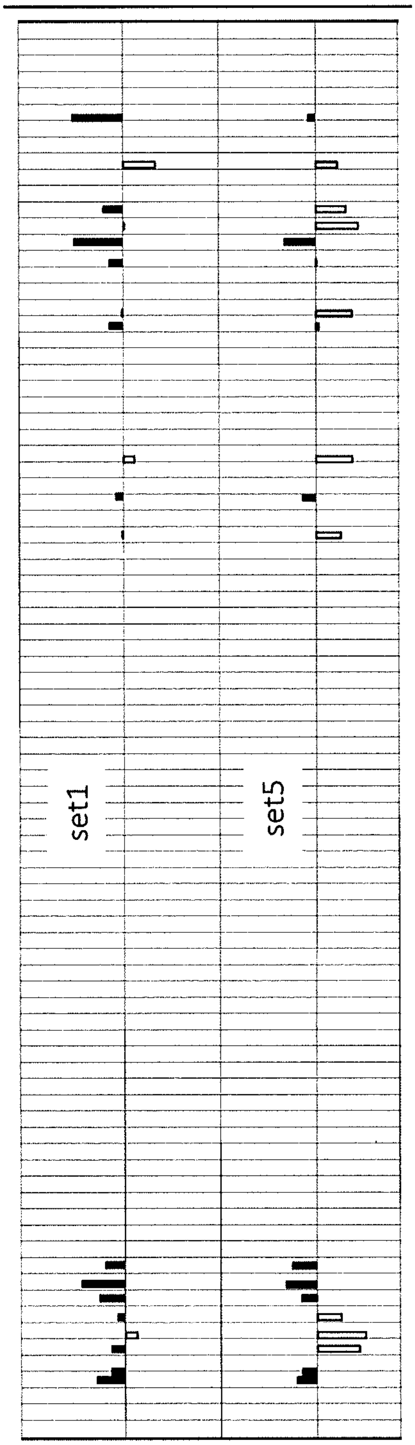

Figure 9

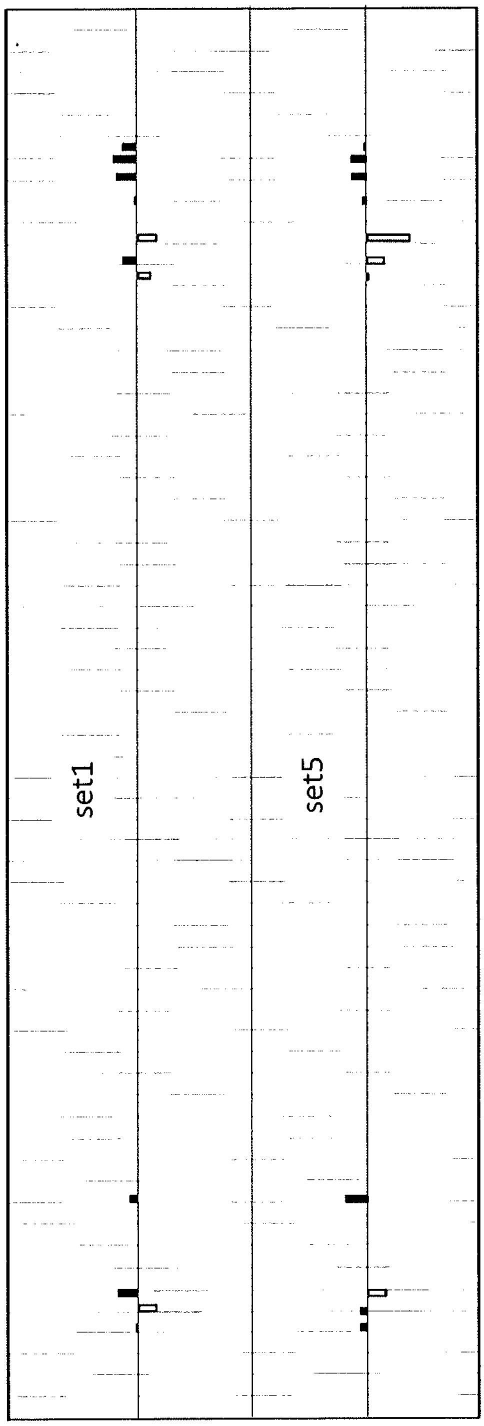

# Figure 10

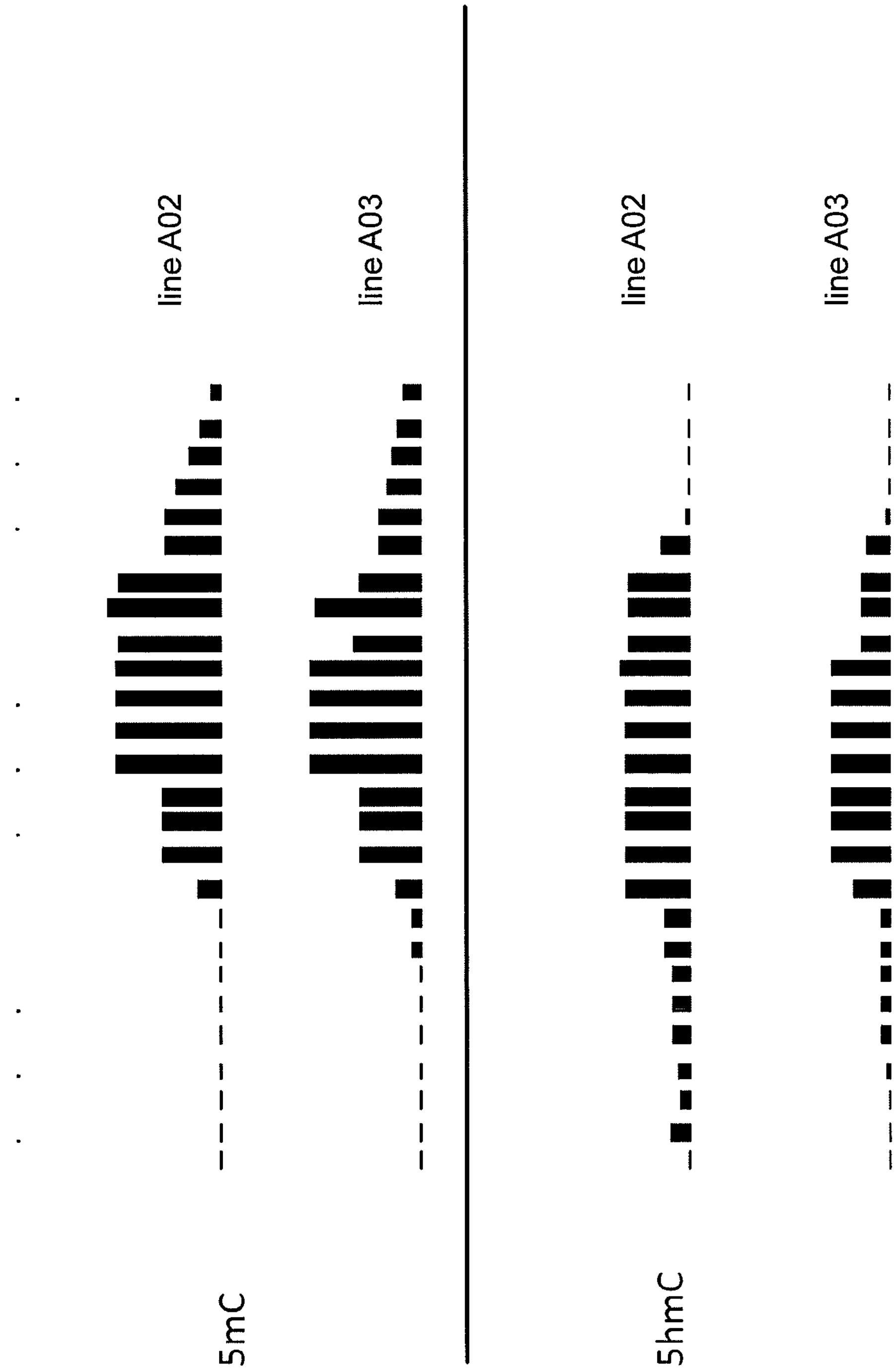


Figure 10: Methylation and hydroxymethylation patterns of human ES cells

# Figure 11

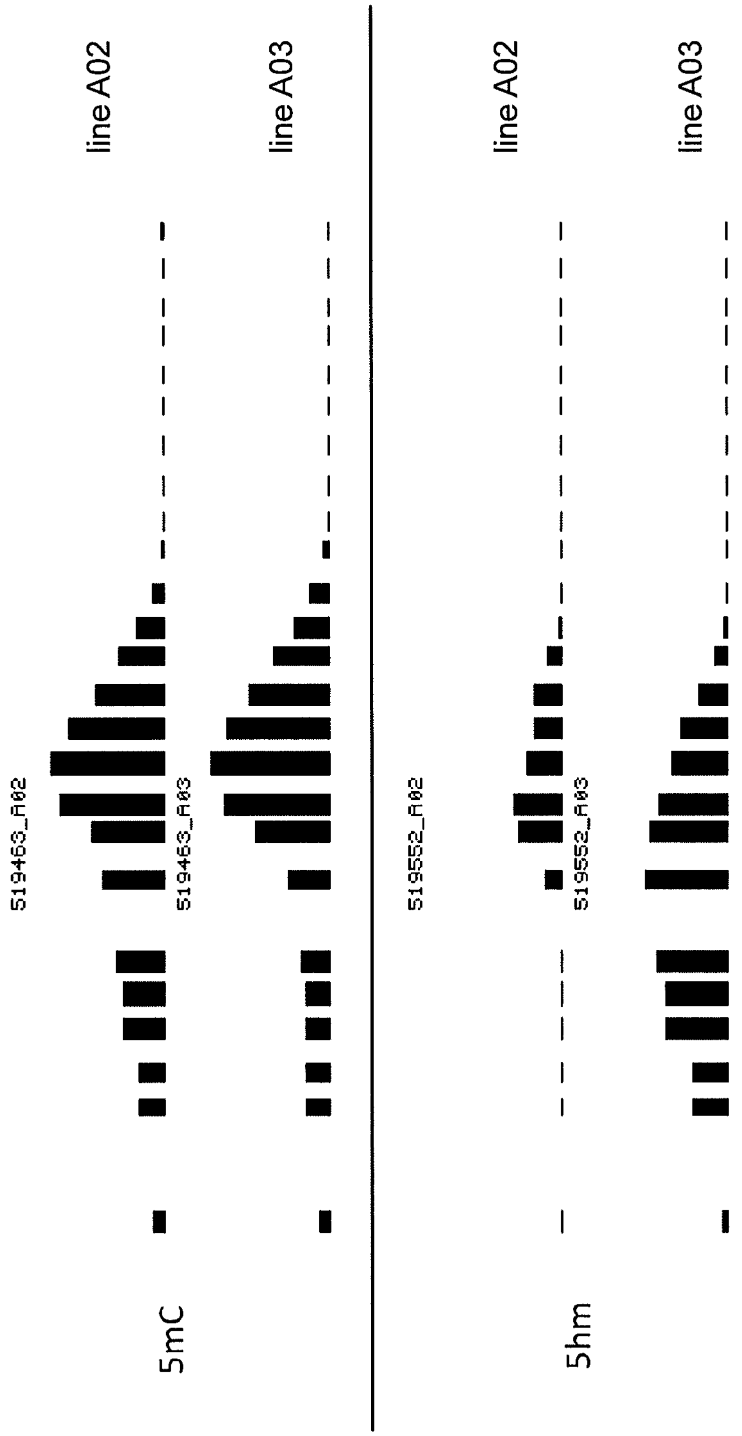


Figure 11: Methylation and hydroxymethylation patterns of human ES cells

# Figure 12

Figure 12: Methylation and hydroxymethylation patterns of human ES cells

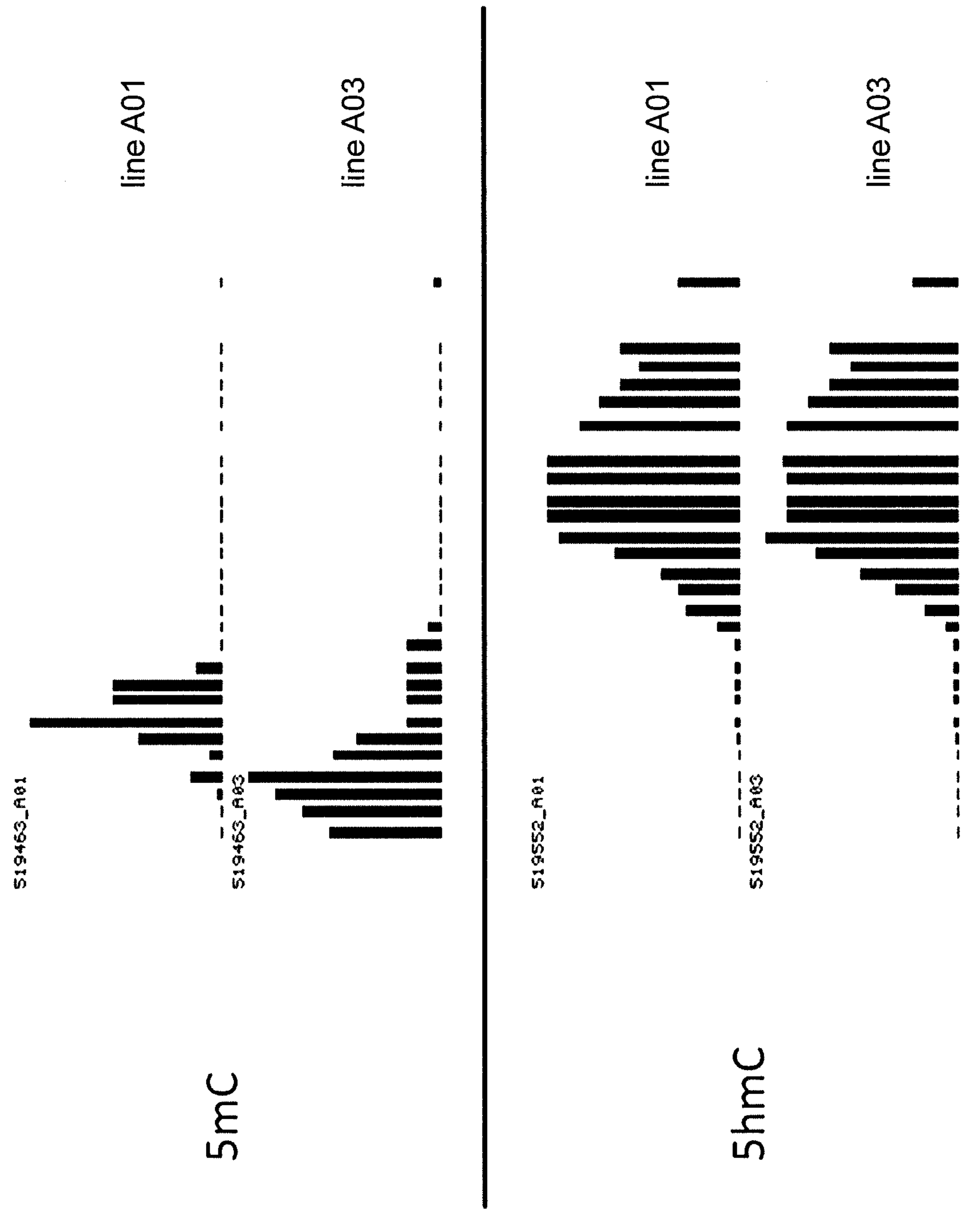

# Figure 13

Figure 13: Methylation and hydroxymethylation patterns of human ES cells

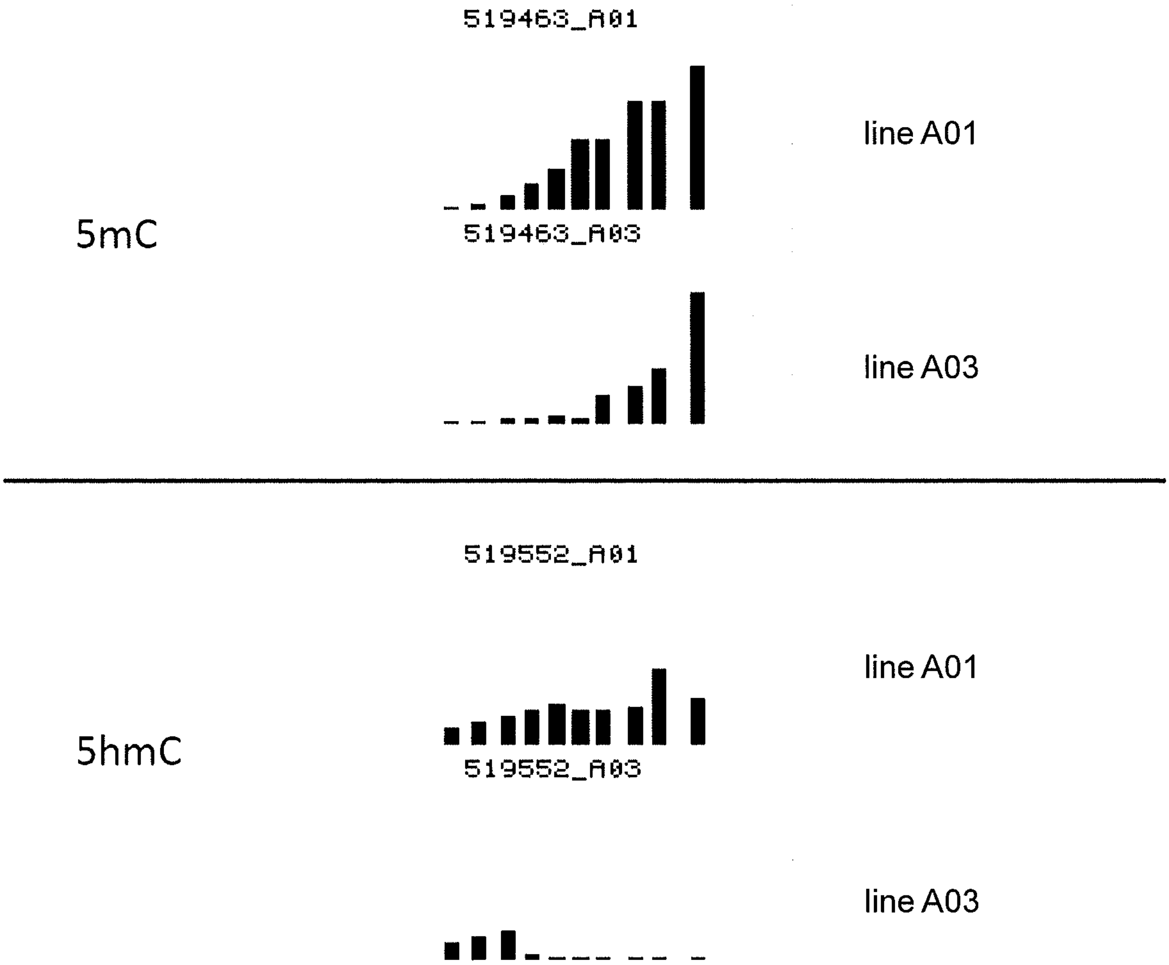

# Figure 14

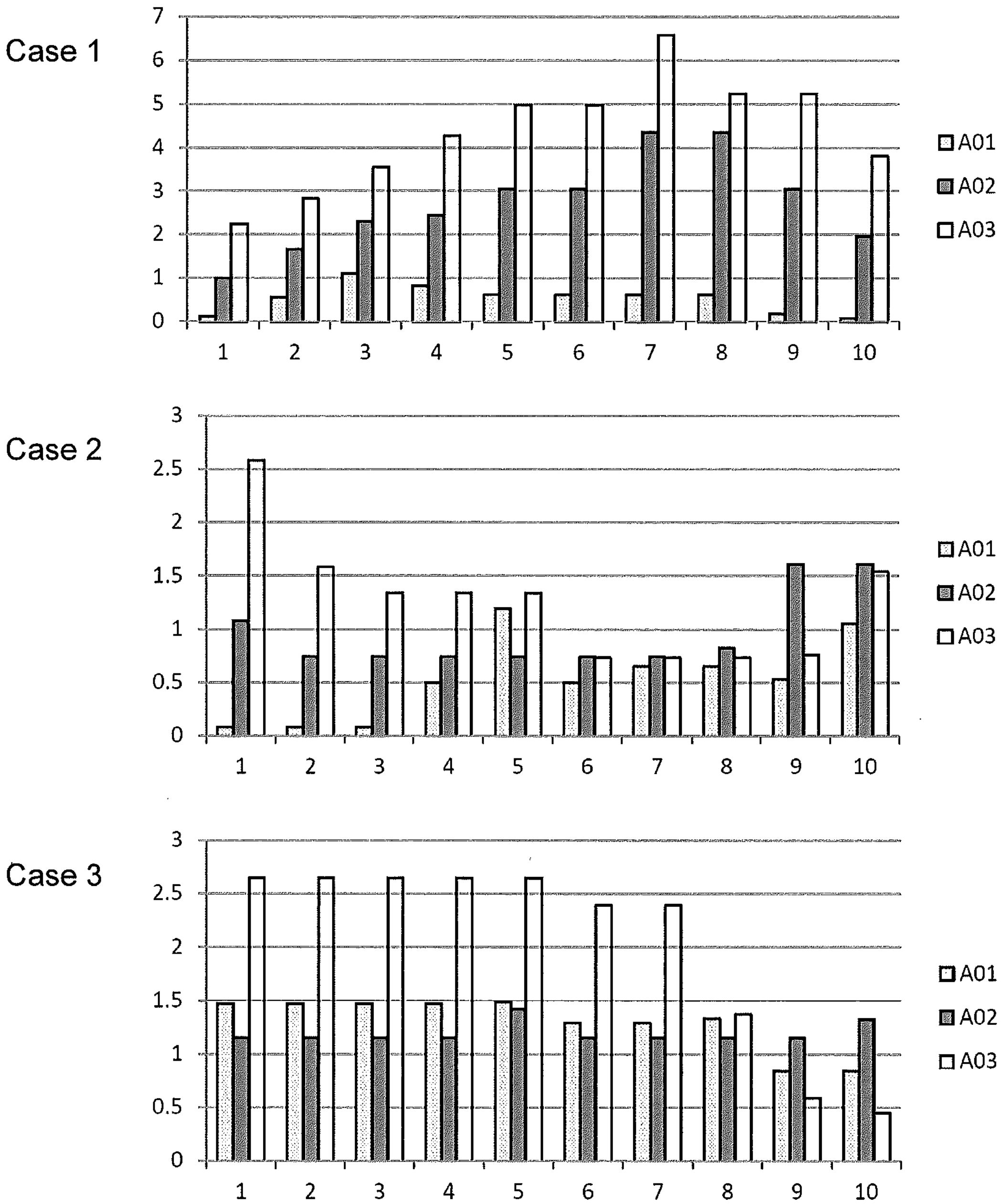

# Figure 15

| Distance | Probe Start | Probe End | A01 | A02 | A03 | |
|---|---|---|---|---|---|---|
| 0 | 211209 | 211266 | 0.12 | 0.99 | 2.25 | |
| 124 | 211333 | 211382 | 0.56 | 1.65 | 2.82 | |
| 222 | 211431 | 211492 | 1.09 | 2.3 | 3.54 | |
| 306 | 211515 | 211570 | 0.82 | 2.44 | 4.26 | |
| 424 | 211633 | 211684 | 0.61 | 3.05 | 4.97 | ← Framed box of Case 1 |
| 500 | 211709 | 211772 | 0.61 | 3.05 | 4.97 | |
| 604 | 211813 | 211869 | 0.61 | 4.35 | 6.58 | |
| 702 | 211911 | 211960 | 0.61 | 4.35 | 5.23 | |
| 824 | 212033 | 212085 | 0.18 | 3.05 | 5.23 | |
| 902 | 212111 | 212160 | 0.08 | 1.96 | 3.8 | |
| 1024 | 212233 | 212293 | 0.08 | 1.08 | 2.58 | |
| 1110 | 212319 | 212368 | 0.08 | 0.74 | 1.58 | |
| 1224 | 212433 | 212495 | 0.08 | 0.74 | 1.34 | |
| 1306 | 212515 | 212564 | 0.5 | 0.74 | 1.34 | |
| 1400 | 212609 | 212665 | 1.19 | 0.74 | 1.34 | ← Framed box of Case 2 |
| 1500 | 212709 | 212760 | 0.5 | 0.74 | 0.73 | |
| 1625 | 212834 | 212895 | 0.65 | 0.74 | 0.73 | |
| 1725 | 212934 | 212987 | 0.65 | 0.82 | 0.73 | |
| 1801 | 213010 | 213072 | 0.53 | 1.61 | 0.76 | |
| 1911 | 213120 | 213169 | 1.05 | 1.61 | 1.54 | |
| 2025 | 213234 | 213283 | 1.05 | 1.61 | 1.63 | |
| 2117 | 213326 | 213375 | 1.05 | 1.61 | 1.63 | |
| 2201 | 213410 | 213459 | 0.71 | 1.61 | 1.63 | |
| 2301 | 213510 | 213559 | 0.71 | 1.61 | 1.63 | |
| 2403 | 213612 | 213661 | 1.47 | 1.15 | 2.65 | |
| 2502 | 213711 | 213760 | 1.47 | 1.15 | 2.65 | |
| 2600 | 213809 | 213858 | 1.47 | 1.15 | 2.65 | |
| 2725 | 213934 | 213983 | 1.47 | 1.15 | 2.65 | |
| 2817 | 214026 | 214075 | 1.49 | 1.42 | 2.65 | ← Framed box of Case 3 |
| 2917 | 214126 | 214175 | 1.29 | 1.15 | 2.39 | |
| 3011 | 214220 | 214269 | 1.29 | 1.15 | 2.39 | |
| 3113 | 214322 | 214371 | 1.33 | 1.15 | 1.37 | |
| 3215 | 214424 | 214473 | 0.84 | 1.15 | 0.59 | |
| 3357 | 214566 | 214615 | 0.84 | 1.32 | 0.45 | |
| 3451 | 214660 | 214709 | 0.84 | 1.32 | 0.45 | |
| 3575 | 214784 | 214833 | 0.71 | 1.13 | 0.29 | |
| 3667 | 214876 | 214925 | 0.58 | 0.94 | 0.15 | |
| 3751 | 214960 | 215009 | 0.58 | 0.94 | 0.15 | |
| 3857 | 215066 | 215115 | 0.44 | 0.75 | 0.04 | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012037456 A
- JP 2013005594 A
- JP 2009165478 A


### Non-patent literature cited in the description


- *Nature Biotechnology,* 2007, vol. 25 (7), 803-816
- **JAMES R. TOLLERVEY et al.** *Epigenetics,* 2012, vol. 7, 823-840
- **KOICHIRO NISHINO et al.** *PLoS one,* 2010, vol. 5, e13017
- **JULIA ARAND et al.** *PLoS one,* 2012, vol. 8, e1002750
- **COLM NESTOR et al.** *BioTechnique,* 2010, vol. 48, 317-319
- **YUN HUANG et al.** *PLoS one,* 2010, vol. 5, e8888
- **LI N et al.** *Methods,* 2010, vol. 52, 203-212
- **JOSHUA D. TOMPKINS et al.** *PNAS,* 2012, vol. 109, 12544-12549
- **STRELCHENKO N et al.** *Reprod Biomed Online.,* 2004, vol. 9, 623-629
- **KLIMANSKAYA I et al.** *Nature,* 2006, vol. 444, 481-485
- **CHUNG Y et al.** *Cell Stem Cell,* 2008, vol. 2, 113-117
- **TAKAHASHI, K. et al.** *Cell,* 30 November 2007, vol. 131, 861-872
- **THOMSON, J.A. et al.** *Science,* 06 November 1998, vol. 282, 1145-1147
- **TAKAHASHI K et al.** *Cell,* 2006, vol. 126, 663-676
- **TAKAHASHI K et al.** *Cell,* 2007, vol. 131, 861-872
- **SCACHERI et al.** *Methods Enzymol.,* 2006, vol. 411, 270-82
- **RAKYAN VK et al.** *Genome Res,* 2008, vol. 18, 1518-1529
- **NICOL JW et al.** *Bioinformatics.,* 04 August 2009
- **HELT GA et al.** *BMC Bioinformatics.,* 25 August 2009, vol. 10 (1), 266